(19)

(11) **EP 3 529 622 B1**

(12) **EUROPEAN PATENT SPECIFICATION**

(45) Date of publication and mention
of the grant of the patent:
**12.03.2025 Bulletin 2025/11**

(21) Application number: **17862400.3**

(22) Date of filing: **18.10.2017**

(51) International Patent Classification (IPC):
***G01N 33/50*** *(2006.01)*

(52) Cooperative Patent Classification (CPC):
**G01N 33/5023; G01N 33/84;** G01N 2510/00

(86) International application number:
**PCT/US2017/057153**

(87) International publication number:
**WO 2018/075623 (26.04.2018 Gazette 2018/17)**

(54) **GENETICALLY ENCODED CELL DEATH INDICATORS AND METHODS OF USE**

GENETISCH CODIERTE ZELLTODINDIKATOREN UND VERFAHREN ZUR VERWENDUNG

INDICATEURS DE MORT CELLULAIRE GÉNÉTIQUEMENT CODÉS ET LEURS MÉTHODES
D'UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.10.2016 US 201662409941 P**

(43) Date of publication of application:
**28.08.2019 Bulletin 2019/35**

(73) Proprietor: **The J. David Gladstone Institutes, A
Testamentary
Trust Established under The Will of
J. David Gladstone
San Francisco, California 94158 (US)**

(72) Inventors:
- **LINSLEY, Jeremy
San Francisco
CA 94158 (US)**
- **SHAH, Kevan
San Francisco
CA 94158 (US)**
- **FINKBEINER, Steven, M.
San Francisco
CA 94158 (US)**

(74) Representative: **Reddie & Grose LLP
The White Chapel Building
10 Whitechapel High Street
London E1 8QS (GB)**

(56) References cited:
US-A1- 2014 101 785    US-A1- 2015 226 755
US-A1- 2016 176 931

- BADURA ALEKSANDRA ET AL: "Fast calcium
sensor proteins for monitoring neural activity",
NEUROPHOTONICS, SOCIETY OF PHOTO-
OPTICAL INSTRUMENTATION ENGINEERS, 1000
20TH ST. BELLINGHAM WA 98225-6705 USA, vol.
1, no. 2, 1 October 2014 (2014-10-01), pages
25008, XP060048048, ISSN: 2329-423X, [retrieved
on 20141017], DOI: 10.1117/1.NPH.1.2.025008
- CHANG YAO-CHUAN ET AL: "In vivo
characterization of genetic expression of virus-
transduced calcium indicators in retinal ganglion
cells using a low-cost funduscope", 2016 38TH
ANNUAL INTERNATIONAL CONFERENCE OF
THE IEEE ENGINEERING IN MEDICINE AND
BIOLOGY SOCIETY (EMBC), IEEE, 16 August
2016 (2016-08-16), pages 1316 - 1319,
XP032979401, DOI: 10.1109/EMBC.2016.7590949
- ANONYMOUS: "pAAV-hSyn1-mRuby2-GSG-P2A-
GCaMP6f-WPRE-pA", 18 August 2015
(2015-08-18), pages 1 - 4, XP055598675,
Retrieved from the Internet <URL:https://web.
archive.org/web/20150818042402/http://www.
addgene.org/50943>

EP 3 529 622 B1

**(Cont. next page)**

- **MCLEAN ET AL.: "Widespread neuron-specific transgene expression in brain and spinal cord following synapsin promoter-driven AAV9 neonatal intracerebroventricular injection", NEUROSCI LETT., vol. 576, 2014, pages 73 - 78, XP028877633**
- **SUZUKI ET AL.: "Imaging intraorganellar Ca2+ at subcellular resolution using CEPIA", NAT COMMUN., vol. 5, 2014, pages 4153, XP055481740**

Remarks:
   The complete document including Reference Table(s) and the Sequence Listing(s) can be downloaded from the EPO website

Remarks:
   The complete document including Reference Table(s) and the Sequence Listing(s) can be downloaded from the EPO website

**Description**

PRIORITY CLAIM

This application claims priority from U.S. Provisional Application 62/409,941, filed October 19, 2016.

FIELD

The disclosure relates to genetically encoded death indicator (GEDI) polypeptides and nucleic acid molecules encoding such polypeptides. In addition, the disclosure also provides methods of using such nucleic acids and polypeptides to monitor cell death events in vitro and in vivo, particularly in neuronal cell death.

BACKGROUND

Calcium ions ($Ca^{2+}$) are involved in many signaling processes in a broad range of cells, tissues and organisms including, for example, muscle contraction, nerve signaling, fertilization and cell division. Among these, calcium transients have been monitored in the field of neuroscience as an indication of neuronal activity and/or health. Action potentials (APs) trigger large spikes in intracellular calcium through voltage-sensitive calcium channels. Genetically-encoded calcium indicators (GECIs) are widely used for imaging these calcium fluxes. Importantly, GECIs are engineered to fluoresce with a dynamic range based on physiological levels $Ca^{2+}$ within healthy cells.

$Ca^{2+}$ overload has also been associated with cell viability or cell death. In many if not all cell types, the ability to maintain a gradient of $Ca^{2+}$ between the inside and outside of the cell is essential to life - once $Ca^{2+}$ levels inside the cell are equivalent to that outside, the cell can be classified as dead.

A pAAV-hSyn1-mRuby2-GSG-P2A-GCaMP6f-WPRE-pA vector is disclosed at https://web.archive.org/web/20150818042402/http://www.addgene.org/50943/.

US2015/0226755 discloses methods of selectively labeling cells by introduction of circularly permutated GCaMP molecules which exhibit photoconversion from green to red fluorescence when exposed to blue light.

Suzuki et al.: "Imaging intraorganellar Ca2+ at subcellular resolution using CEPIA" Nat. Commun., vol. 5, 2014, page 4153 discloses a family of genetically encoded $Ca^{2+}$ indicators, named calcium-measuring organelle-entrapped protein indicators (CEPIA), which can be utilized for intraorganellar $Ca^{2+}$ imaging.

SUMMARY

Methods and materials are provided for detecting and monitoring cell death, for example, neuronal cell death.

In various embodiments, the present invention provides isolated nucleic acids as defined in claim 1..

In various embodiments, the present invention also provides vectors, isolated cells, non-human animals, and/or organotypic slice cultures as defined in claims 5, 7, 8 and 9.

In various embodiments, the present invention also provides methods for monitoring calcium flux in a cell as defined in claim 10.

In various embodiments, the present invention also provides methods for monitoring cell death in a cell as defined in claim 11.

In various embodiments, the present invention also provides methods of screening for an agent that is capable of causing an intracellular calcium concentration fluctuation in a cell, as defined in claim 12.

In some embodiments, which may be combined with any of the above or below embodiments, the promoter is a tissue specific promoter, for example, a neuron specific promoter.

In some embodiments, which may be combined with any of the above or below embodiments, the first detectable marker, the second detectable marker, or both comprise a fluorescent label, for example, green fluorescent protein (GFP), mApple, mTagBFP, or variants thereof.

According to the invention, the first detectable marker and the second detectable marker are distinguishable from each other.

In some aspects, which may be combined with any of the above or below aspects, the modified calcium binding motif comprises at least one genetic modification.

According to the invention, the modified calcium binding motif comprises at least one amino acid substitution of SEQ ID NO. 1 or variants thereof selected from the group consisting of E31D, F92W, E104D, D133E, or any combination thereof.

In some embodiments, which may be combined with any of the above or below embodiments, the modified calcium binding motif has a calcium dissociation constant ($K_d$) greater than 1 $\mu$M. In other embodiments, which may be combined with any of the above or below embodiments, the modified calcium binding motif has a $K_d$ greater than 100 $\mu$M.

In some embodiments, which may be combined with any of the above or below aspects, the nucleic acid sequence does not comprise a localization sequence. According to the invention the nucleic acid sequence does not comprise a nuclear localization sequence, an endoplasmic reticulum localization sequence, a mitochondrial localization sequence, or any combination thereof.

In some embodiments, which may be combined with any of the above or below embodiments, the vector is an adenovirus, adeno-associated virus (AAV), lentivirus, retrovirus, or episomal vector.

In some embodiments, which may be combined with any of the above or below embodiments, the cell is a neural cell, a muscle cell, a cancer cell, or a gamete. In some embodiments, which may be combined with any of the above or below embodiments, the cell is a human cell, for example, a human induced pluripotent stem cell (iPSC).

In some embodiments, which may be combined with any of the above or below embodiments, at least one of the method steps are performed using confocal microscopy.

In some embodiments, which may be combined with any of the above or below embodiments, the method is a high-throughput method. In some embodiments, which may be combined with any of the above or below embodiments, the method is an automated method.

In some embodiments, which may be combined with any of the above or below embodiments, a signal intensity ratio of greater than 0.05 indicates the cell is dead.

In some embodiments, which may be combined with any of the above or below embodiments, cell death comprises apoptosis, necrosis, autophagy, necroptosis, or any combination thereof.

In some embodiments, which may be combined with any of the above or below embodiments, cell death is determined when $Ca^{2+}$ levels inside the cell are equivalent to $Ca^{2+}$ levels outside the cell.

DESCRIPTION OF THE DRAWINGS

FIG. 1A are representative images of rat primary cortical neurons expressing GCaMP6f, mRuby, and HttEx1-Q97 and demonstrate that GCaMP6f signal increases in neurons as they die.

FIG. 1B shows the ratios of GCaMP6f/mRuby signal from rat primary cortical neurons expressing HttEx1-Q97 and HttEx1-Q25 at 0, 24, 48, 72, 96, and 120 hours post transfection, and a line at 0.75 separating dead from live neurons demonstrating live and dead neurons can be segregated across time points using the GCaMP6f/mRuby signal.

FIG. 1C shows a cumulative hazard plot derived from the data in FIG 1b using the GCaMP6f/mRuby signal to distinguish live and dead neurons showing HttEx1-Q97 is more toxic than HttEx1-Q25 as has been previously been described by Arrasate M. et al. (2014) Nature 431:805-810

FIG. 2A shows representative time lapse images of GCaMP6f (top), and RGEDI (bottom) at baseline, after 10Hz voltage stimulation, and after NaAz exposure to induce death showing RGEDI responds only at death of the neuron and not during voltage stimulation.

FIG. 2B shows a representative trace of ΔF/F GCaMP6f/mRuby during voltage stimulation, and after NaAz exposure.

FIG. 2C shows a representative trace of ΔF/F RGEDI/EGFP during voltage stimulation, and after NaAz exposure.

FIG. 2D shows that the ratio of GCaMP6f response to voltage stimulation over death is significantly increased compared to RGEDI.

FIG. 2E are time lapse representative images of rat primary cortical neurons expressing RGEDI-P2a-EGFP at 0, 6, and 21 hours post transfection with the left neuron (1) dying and the right neuron (2) surviving.

FIG. 2F shows quantification of the ΔF/F RGEDI/EGFP ratio of neurons from FIG2E from 0 to 21 hours after transfection showing the ratio increases in the dead neuron 1 but does not in neuron 2.

FIG. 2G shows regressions of RGEDI and EGFP fluorescence decay from 642 neurons, demonstrating fluorescence decay after death of RGEDI and EGFP signals proceed at the same rate.

FIG. 3A are representative images of EGFP morphology signals of rat primary cortical neurons at 0, 30, 60, 300, and 600 seconds after $NaN_3$ exposure which induces acute death, showing acute changes in morphology associated with neuronal death.

**FIG. 3B** are representative images of RGEDI signal of same rat primary cortical neurons from FIG. 3A at 0, 30, 60, 300, and 600 seconds after $NaN_3$ exposure showing an increase in RGEDI signal during death that precedes changes in morphology.

**FIG. 3C** shows quantification of the log RGEDI/EGFP signal of rat primary cortical neurons 0, 30, 60, 300, and 600 seconds after $NaN_3$ exposure in media with and without $Ca^{2+}$ compared to neurons after NaCl exposure showing the increase in RGEDI/EGFP ratio during death $NaN_3$ induced death is primarily due to influx of extracellular $Ca^{2+}$. ANOVA Tukey's ***$p<0.001$, ns = not significant.

**FIG. 4A** is a schematic representation of a modified GECI construct according to an embodiment of the present disclosure.

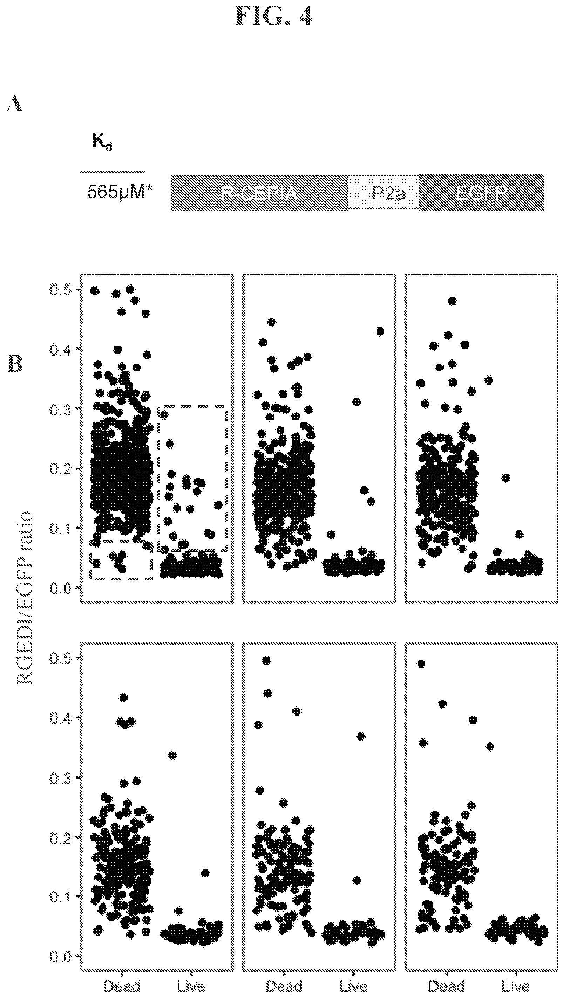

**FIG. 4B** demonstrates a modified GECI construct according to an embodiment of the present disclosure is capable of distinguishing live from dead neurons in culture. The modified GECI (RGEDI-P2A-EGFP) substantially improves live/dead labeling compared to hSyn1:GCaMP6f-P2a-mRuby. The ratio RGEDI to EGFP was quantified for each automatically segmented neuron and classified as either dead or alive based on its morphology. Checkered boxes contain neurons in which it was unclear on first pass of classification by morphology if the neuron was dead or alive. Upon second examination, each neuron clearly misclassified at first, or is found to be dead in the subsequent time point (top box) or alive in the subsequent time point (bottom box).

**FIG. 5** provides an example analysis summary and shows that use of robotic microscopy with RGEDI-P2A-EGFP followed by fully automated analysis provides comparable hazard ratios and statistical outcome measures for the same data set analyzed manually.

**FIG. 6A** shows rat primary cortical neurons transfected with either HttEx1-Q97, TDP43, or α-synuclein at 0 hours post transfection and all show increases in RGEDI signal upon death at 24 hours.

**FIG. 6B** shows the GEDI ratios derived from neurons expressing HttEx1-Q97, HttEx1-Q25, TDP43, or α-synuclein compared to control neurons in high throughput using automated imaging with a GEDI threshold set at 0.5 dividing dead and live neurons over 8 time points.

**FIG. 6C** shows an automated cumulative hazard plot derived from data in FIG. 6B showing HttEx1-Q97, TDP43, and α-synuclein each have increased toxicity over time compared to control, indicating GEDI can be used on multiple types of neurodegenerative diseases.

**FIG. 7A** are representative images from GEDI expressed in human patient derived motor neurons from control or induced pluripotent stem cells expressing containing the SOD1-D90A mutation at 0, 12, 24, 36, 48, 60, 72, and 84 hours after transfection showing that GEDI reports neuronal death in human neurons.

**FIG. 7B** shows the automated GEDI ratio quantification of neurons over 156 hours post transfection with a derived GEDI threshold of 0.05 that distinguishes dead from live neurons.

**FIG. 7C** shows an automated cumulative hazard plot derived from data in FIG. 7B showing human neurons containing the SOD1-D90A mutation have a higher risk of death than control neurons.

**FIG. 8A** is a schematic representation of several modified GCaMP constructs according to embodiments of the present disclosure and representation of the intensity/spectrum changes of the proteins in response to $Ca^{2+}$ concentrations. The complementary attached morphology marker is included on the right. These modified GCaMP constructs are optimized to fluoresce at extracellular $Ca^{2+}$ levels.

**FIG. 8B** summarizes features of several modified GCaMP constructs according to embodiments of the present disclosure including color combinations along with predicted Hill Coefficients and $K_d$ for $Ca^{2+}$ binding.

**FIG. 9A** shows representative images of rat primary cortical neurons at 0, 5 and 10 minutes after exposure to NaAz when expressing GCaMP-P2a-mRuby. GEDI ratio time lapse response to neurons at 0, 5, 10, 15, and 20 minutes after NaAz is quantified on the right of the time lapse image showing the GEDI ratio responds reproducibly and acutely to death with the GCaMP-P2a-mRuby variant.

**FIG. 9B** shows representative images of rat primary cortical neurons at 0, 5 and 10 minutes after exposure to NaAz when expressing RGEDI-P2a-EGFP. GEDI ratio time lapse response to neurons at 0, 5, 10, 15, and 20 minutes after NaAz is quantified on the right of the time lapse image showing the GEDI ratio responds reproducibly and acutely to death with the RGEDI-P2a-EGFP variant.

**FIG. 9C** shows representative images of rat primary cortical neurons at 0, 5 and 10 minutes after exposure to NaAz when expressing RGEDI-P2a-3xTagBFP2. GEDI ratio time lapse response to neurons at 0, 5, 10, 15, and 20 minutes after NaAz is quantified on the right of the time lapse image showing the GEDI ratio responds reproducibly and acutely to death with the RGEDI-P2a-3xTagBFP2 variant.

**FIG. 9D** shows representative images of rat primary cortical neurons at 0, 5 and 10 minutes after exposure to NaAz when expressing RGEDInls-P2a-EGFPnls. GEDI ratio time lapse response to neurons at 0, 5, 10, 15, and 20 minutes after NaAz is quantified on the right of the time lapse image showing the GEDI ratio responds reproducibly and acutely to death with the RGEDInls-P2a-EGFPnls variant.

**FIG. 10A** shows representative images of experiments in which RGEDI-P2a-tag3xBFP was co-transfected with the independent green channel mitochondrial marker mitoGFP biosensor recorded in parallel with the GEDI death indicator at 0, 24, 48 and 72 hours post transfection. Time of neuronal death is indicated with a white asterisk.

**FIG. 10B** shows representative images of experiments in which RGEDI-P2a-tag3xBFP was co-transfected with the independent green channel lysosomal marker GFP-TrpML1 biosensor recorded in parallel with the GEDI death indicator at 0, 24, 48 and 72 hours post transfection. Time of neuronal death is indicated with a white asterisk.

**FIG. 10C** shows representative images of experiments in which RGEDI-P2a-tag3xBFP was co-transfected with the independent green channel cytosolic $Ca^{2+}$ indicator biosensor recorded in parallel with the GEDI death indicator at 0, 24, 48 and 72 hours post transfection. Time of neuronal death is indicated with a white asterisk.

**FIG. 11A** shows the coexpression of GCaMP7 and NTR-mCherry in motor neurons within a live zebrafish larvae. NTR-mCherry expression in neurons can cause neuronal death when exposed to MTZ but not DMSO.

**FIG. 11B** shows representative images of GCaMP7 fluorescence increase in spinal motor neurons at 0 and after 24 hours of MTZ exposure not in DMSO treated zebrafish.

**FIG. 11C** shows the calculated GEDI ratio using GCaMP7 and mCherry of motor neurons at 0, 24, and 48 hours after MTZ treatment showing the GEDI ratio increases indicating neuronal death in motor neurons incubated in MTZ but not in DMSO, establishing a GEDI threshold of 0.2 within the live zebrafish.

**FIG. 12A** shows a survival plot of rat primary cortical neurons after exposure to 0.05mM, 0.1mM, or 1mM Glutamate (Glu) derived using GEDI ratio showing a subset of neurons are resistant to Glutamate, and a subset of neurons has increased sensitivity to Glutamate.

**FIG. 12B** shows representative images of EGFP and RGEDI prior to Glutamate exposure, and 3 hours after Glutamate exposure of neurons that are resistant (top) and sensitive to Glutamate (bottom).

**FIG. 12C** is the mean quantification of neurite area of neurons prior to 1mM Glutamate exposure showing neurons that survive Glutamate exposure have significantly more neurite area before exposure than those that do not survive.

**FIG. 13A** contains representative single zebrafish neurons within a live zebrafish brain co-expressing RGEDI-P2a-3xtagBFP and NTR-mVenus at 0, 24, and 48 hours after application of either DMSO (top), or MTZ (bottom) showing RGEDI signal increases during neuronal death.

**FIG. 13B** contains representative a single zebrafish skeletal muscle fiber co-expressing RGEDI-P2a-3xtagBFP and NTR-mVenus at 0, 24, and 48 hours showing RGEDI signal increases during skeletal muscle fiber death.

**FIG. 14A** shows representative mouse hippocampal organotypic slice culture co-expressing RGEDI-P2a-EGFP and HttEx1-Q25 (top) or RGEDI-P2a-EGFP and HttEx1-Q97 (bottom), showing elevated RGEDI signal indicating neuronal death in neurons in the HttEx1-Q97 expressing neurons. Right panels are blowups of yellow boxes on left.

**FIG. 14B** shows quantification of GEDI ratio in neurons within slices transfected with HttEx1-Q97 is significantly higher than in slices transfected with HttEx1-Q25 indicating GEDI distinguishes the higher toxicity of. HttEx1-Q97 expression in neurons..

**FIG. 15** provides a list of various amino acid substitutions to calmodulin domain and binding affinity.

**FIG. 16** demonstrates that pseudo-ratiometric GCaMP-P2A-mRuby construct is capable of distinguishing live from dead neurons in vitro. The ratio of GCaMP6f to mRuby was quantified for each automatically segmented neuron and classified as either dead or alive based on morphology. Fluctuations in GCaMP fluorescence blur the separation between live and dead GCaMP.

**FIG. 17A** depicts representative images of rat primary cortical neurons showing fluctuations in $Ca^{2+}$ concentration visualized by GCaMP6f-P2A-mRuby expression over a course of 5 days.

**FIG. 17B** shows the GCAMP $\Delta$F/F responses in rat primary cortical neuron culture for the individual neurons labeled in FIG. 2A at 0 hrs, 24 hrs, 48 hrs, 72 hrs, 96 hrs, and 120 hrs.

**FIG. 18A** is a representative image of rat primary cortical neurons expressing the amino-terminal exon 1 fragment of huntingtin (HttEx1) containing polyQ$^{97}$ that results in polyQ-expansion-dependent, neuron-specific cell death at time 0 of longitudinal tracking of single neurons.

**FIG. 18B** is a representative image of the same neurons shown in FIG. 18A 24 hours later.

**FIG. 18C** demonstrates the ability of GCaMP6 and mRuby signal to discriminate live and dead neurons from a population of neurons expressing HttEx1 polyQ$^{97}$ or HttEx1 polyQ$^{25}$ at 0 hrs, 24 hrs, 48 hrs, 72 hrs, 96 hrs, and 120 hrs.

**FIG. 19A** shows acute GCaMP6 signal increase in a dying motor neuron derived from human pluripotent stem cells with the G298S mutation in TDP-43 that causes Amyotrophic Lateral Sclerosis (ALS) in representative images from 0 hours, 2.5 hours, and 7 hours in representative images.

**FIG. 19B** shows acute RGEDI signal increase in motor neurons derived from human pluripotent stem cells in dying motor neurons derived from human pluripotent stem cells with the G298S mutation in TDP-43 in representative images from 0 hours, 2.5 hours, and 7 hours.

**FIG. 20A** demonstrates that GCaMP expression increases in dying motor neurons of zebrafish larva in time lapse imaging after targeted neuronal ablation with metrodinazole (MTZ). n = 13 neurons MTZ, 16 neurons DMSO. 2 way ANOVA * p<0.05, **** p<0.0001.

**FIG. 20B** demonstrates that GCaMP signal is chronically increased during neurodegeneration *in vivo.*

**FIG. 21A** depicts representative images of dying rat primary cortical neurons expressing HttEx1 polyQ$^{97}$ showing fluctuations in $Ca^{2+}$ concentration visualized by RGEDI-P2A-EGFP (GEDI) expression over a course of 5 days.

**FIG. 21B** shows a zoomed in image of the 120 hrs time point image from FIG. 21A.

**FIG. 21C** shows the GEDI $\Delta$F/F responses in rat primary cortical neuron culture for the individual neurons labeled in FIG. 21A at 0 hrs, 24 hrs, 48 hrs, 72 hrs, 96 hrs, and 120 hrs.

**FIG. 21D** shows treatment of neurons with 10 mM KCl failed to excite GEDI signal.

**FIG. 22** demonstrates the GEDI constructs can also be used to label neuronal cell death in organotypic slice cultures.

DETAILED DESCRIPTION

After reading this description it will become apparent to one skilled in the art how to implement the invention in various alternative embodiments and alternative applications. However, all the various embodiments of the present invention will not be described herein. It will be understood that the embodiments presented here are presented by way of example only, and not limitation.

The detailed description is divided into various sections only for the reader's convenience and disclosure found in any section may be combined with that in another section.

The practice of the present disclosure will employ, unless otherwise indicated, conventional techniques of tissue culture, immunology, molecular biology, cell biology and recombinant DNA, which are within the skill of the art. See, e.g., Sambrook and Russell eds. (2001) Molecular Cloning: A Laboratory Manual, 3rd edition; the series Ausubel et al. eds. (2007) Current Protocols in Molecular Biology; the series Methods in Enzymology (Academic Press, Inc., N.Y.); MacPherson et al. (1991) PCR 1: A Practical Approach (IRL Press at Oxford University Press); MacPherson et al. (1995) PCR 2: A Practical Approach; Harlow and Lane eds. (1999) Antibodies, A Laboratory Manual; Freshney (2005) Culture of Animal Cells: A Manual of Basic Technique, 5th edition; Gait ed. (1984) Oligonucleotide Synthesis; U.S. Pat. No. 4,683,195; Hames and Higgins eds. (1984) Nucleic Acid Hybridization; Anderson (1999) Nucleic Acid Hybridization; Hames and Higgins eds. (1984) Transcription and Translation; IRL Press (1986) Immobilized Cells and Enzymes; Perbal (1984) A Practical Guide to Molecular Cloning; Miller and Calos eds. (1987) Gene Transfer Vectors for Mammalian Cells (Cold Spring Harbor Laboratory); Makrides ed. (2003) Gene Transfer and Expression in Mammalian Cells; Mayer and Walker eds. (1987) Immunochemical Methods in Cell and Molecular Biology (Academic Press, London); Herzenberg et al. eds (1996) Weir's Handbook of Experimental Immunology; Manipulating the Mouse Embryo: A Laboratory Manual, 3rd edition (2002) Cold Spring Harbor Laboratory Press; Sohail (2004) Gene Silencing by RNA Interference: Technology and Application (CRC Press).

Unless the context indicates otherwise, it is specifically intended that the various features of the invention described herein can be used in any combination. Moreover, the disclosure also contemplates that in some embodiments, any feature or combination of features set forth herein can be excluded or omitted. To illustrate, if the specification states that a complex comprises components A, B and C, it is specifically intended that any of A, B or C, or a combination thereof, can be omitted and disclaimed singularly or in any combination.

All numerical designations, e.g., pH, temperature, time, concentration, and molecular weight, including ranges, are approximations which are varied ( + ) or ( - ) by increments of 1.0 or 0.1, as appropriate, or alternatively by a variation of +/- 15 %, or alternatively 10%, or alternatively 5%, or alternatively 2%. It is to be understood, although not always explicitly stated, that all numerical designations are preceded by the term "about." It is to be understood that such range format is used for convenience and brevity and should be understood flexibly to include numerical values explicitly specified as limits of a range, but also to include all individual numerical values or sub-ranges encompassed within that range as if each numerical value and sub-range is explicitly specified. For example, a ratio in the range of about 1 to about 200 should be understood to include the explicitly recited limits of about 1 and about 200, but also to include individual ratios such as about 2, about 3, and about 4, and sub-ranges such as about 10 to about 50, about 20 to about 100, and so forth. It also is to be understood, although not always explicitly stated, that the reagents described herein are merely exemplary and that equivalents of such are known in the art.

It must be noted that as used herein and in the appended claims, the singular forms "a", "an", and "the" include plural referents unless the context clearly dictates otherwise. Thus, for example, reference to "a cell" includes a plurality of cells.

*Definitions*

As used herein the following terms have the following meanings:

The term "about," as used herein when referring to a measurable value such as an amount or concentration and the like, is meant to encompass variations of 20%, 10%, 5%, 1%, 0.5%, or even 0.1% of the specified amount.

The terms or "acceptable," "effective," or "sufficient" when used to describe the selection of any components, ranges, dose forms, etc. disclosed herein intend that said component, range, dose form, etc. is suitable for the disclosed purpose.

Also as used herein, "and/or" refers to and encompasses any and all possible combinations of one or more of the associated listed items, as well as the lack of combinations when interpreted in the alternative ("or").

"Comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition consisting essentially of the elements as defined herein would not exclude other materials or steps that do not materially affect the basic and novel characteristic(s) of the claimed invention. "Consisting of" shall mean excluding more than trace elements of other ingredients and substantial method steps. Embodiments defined by each of these transition terms are within the scope of this invention.

As used herein, the term "isolated" in reference to a molecule or cell (e.g., an isolated protein, an isolated nucleic acid, or an isolated cell) is one which has been identified and separated and/or recovered from a component of its natural environment. The term "isolated" as used herein with respect to nucleic acids, such as DNA or RNA, refers to molecules separated from other DNAs or RNAs, respectively that are present in the natural source of the macromolecule. The term "isolated nucleic acid" is meant to include nucleic acid fragments which are not naturally occurring as fragments and would not be found in the natural state. The term "isolated" is also used herein to refer to polypeptides, proteins that are isolated from other cellular proteins and is meant to encompass both purified and recombinant polypeptides. In other embodiments, the term "isolated" means separated from constituents, cellular and otherwise, in which the cell, tissue, polynucleotide, peptide, polypeptide, protein, antibody or fragment(s) thereof, which are normally associated in nature. For example, an isolated cell is a cell that is separated form tissue or cells of dissimilar phenotype or genotype. As is apparent to those of skill in the art, a non-naturally occurring polynucleotide, peptide, polypeptide, protein, does not require "isolation" to distinguish it from its naturally occurring counterpart.

The term "modified," "modification," and the like, in reference to a nucleic acid or amino acid sequence refers to any change in one or more nucleic acids and/or amino acids. A non-limiting example of a modification includes mutations. Nucleic acid and/or amino acid mutations include deletions, insertions, and/or substitutions. Such modifications result in the protein encoded by the sequence having altered activity or expression levels within in the cell, for example, the modification may result in an increased or reduced binding affinity of a protein or other signaling molecule.

The term "nucleic acid" refers to two or more deoxyribonucleotides and/or ribonucleotides covalently joined together in either single or double-stranded form.

The term "recombinant nucleic acid" means a nucleic acid of interest that is free of one or more nucleic acids (e.g., genes) which, in the genome occurring in nature of the organism in nature of the organism from which the nucleic acid of interest is derived, flank the nucleic acid of interest. A non-limiting example includes recombinant DNA which is incorporated into the genomic DNA of a prokaryote or eukaryote, or which exists as a separate molecule (e.g., a cDNA, a genomic DNA fragment, or a cDNA fragment produced by PCR or restriction endonuclease digestion) independent of other sequences.

The term "operably linked" refers to a functional linkage between a nucleic acid expression control sequence (e.g., a promoter) and a second nucleic acid sequence, wherein the expression control sequence directs transcription of the nucleic acid corresponding to the second sequence.

As used herein, the term "protein" includes polypeptides, peptides, fragments of proteins, and fusion proteins and refer to a polymeric form of amino acids of any length, which can include genetically coded and non-genetically coded amino acids, chemically or biochemically modified or derivatized amino acids, and polypeptides having modified peptide backbones. The term includes fusion proteins, including, but not limited to, fusion proteins with a heterologous amino acid sequence, fusions with heterologous and homologous leader sequences, with or without N-terminal methionine residues, immunologically tagged proteins, and the like.

The term "sequence identity" with respect to a protein or amino acid sequence (or a DNA or RNA sequence) refers to the percentage of amino acid residues (or nucleotide residues) in a candidate sequence that are identical to the amino acid residues in the specific protein or amino acid sequence (or nucleotide residues in the specific DNA or RNA sequence), after aligning the sequences and introducing gaps, if necessary, to achieve a maximum percent sequence identity, and not considering any conservative substitutions as part of the sequence identity. Alignment can be achieved by any method known to one of skill in the art, for example, by using publicly available programs such as BLAST and EMBOSS. Those skilled in the art can determine appropriate parameters for measuring alignment, including any algorithms needed to achieve maximal alignment over the full length of the sequences being compared.

The term "variant" as used herein, is a nucleic acid or protein that differs from a reference nucleic acid or protein (i.e., calmodulin or fragment thereof), but retains essential properties (i.e., biological activity). A typical variant of a polynucleotide differs in nucleotide sequence from another, reference polynucleotide. Changes in the nucleotide sequence may or may not alter the amino acid sequence of a polypeptide encoded by the reference polynucleotide. Nucleotide changes may result in amino acid substitutions, additions, deletions, fusions, and/or truncations in the polypeptide encoded by the reference sequence.

The term "vector" is used herein to refer to a nucleic acid molecule capable of transferring or transporting another nucleic acid molecule. The transferred nucleic acid is generally linked to, for example, the vector nucleic acid molecule. A vector may include sequences that direct autonomous replication in a cell, or may include sequences sufficient to allow integration into cellular DNA. Useful vectors include, for example, plasmids (e.g., DNA plasmids or RNA plasmids), transposons, cosmids, bacterial or yeast artificial chromosomes and viral vectors. Useful viral vectors include, for example, adenoviruses, retroviruses, particularly replication defective retroviruses, and lentiviruses.

The present disclosure provides novel genetically encoded, calcium-based biosensors for reporting cell death. Rather than detecting a particular pathway of cell death (*e.g.,* apoptosis or necrosis), the genetically encoded death indicators (GEDI) detect the ability of a cell to maintain $Ca^{2+}$ homeostasis. The GEDIs disclosed herein have low toxicity, are well tolerated by cells, and can be used in a variety of *in vitro* and *in vivo* applications including, for example, time-lapse microscopy. These characteristics enable GEDIs to be useful for longitudinal imaging studies of cell death over the course of weeks and months and gives researchers a tool to study the process of cell death (*e.g.,* neurodegeneration) in tissue, cell culture, and *in vivo*.

*Vectors*

Selection of the appropriate vector and promoter for use in the GEDIs of the present disclosure is well within the level of ordinary skill in the art. The expression vector may also contain a ribosome binding site for translation initiation and a transcription terminator. The expression vector may also include appropriate sequences for amplifying expression.

Examples of suitable mammalian expression vectors include, but are not limited to: recombinant viruses, nucleic acid vectors, such as plasmids, bacterial artificial chromosomes, yeast artificial chromosomes, human artificial chromosomes, cDNA, cRNA, and polymerase chain reaction (PCR) product expression cassettes.

Suitable viral vectors include, but are not limited, viral vectors based on retroviruses (including lentiviruses), adenoviruses, adeno-associated viruses, and episomal vectors. An example of a suitable retrovirus-based vector is a vector based on murine moloney leukemia virus (MMLV); however, other recombinant retroviruses may also be used, e.g., Avian Leukosis Virus, Bovine Leukemia Virus, Murine Leukemia Virus (MLV), Mink-Cell focus-Inducing Virus, Murine Sarcoma Virus, Reticuloendotheliosis virus, Gibbon Abe Leukemia Virus, Mason Pfizer Monkey Virus, or Rous Sarcoma Virus (e.g., U.S. Pat. No. 6,333,195).

In other cases, the retrovirus-based vector is a lentivirus-based vector, (e.g., Human Immunodeficiency Virus-1 (HIV-1), Simian Immunodeficiency Virus (SIV) or Feline Immunodeficiency Virus (FIV)). Johnston et al. (1999), Journal of Virology, 73(6):4991-5000 (FIV); Negre et al. (2002) Current Topics in Microbiology and Immunology, 261:53-74 (SIV); Naldini et al. (1996) Science, 272:263-267 (HIV).

The recombinant retrovirus may comprise a viral polypeptide (e.g., retroviral env) to aid entry into the target cell. Such viral polypeptides are well-established in the art, for example, U.S. Pat. No. 5,449,614. The viral polypeptide may be an amphotropic viral polypeptide, for example, amphotropic env, which aids entry into cells derived from multiple species, including cells outside of the original host species. The viral polypeptide may be a xenotropic viral polypeptide that aids entry into cells outside of the original host species. In some embodiments, the viral polypeptide is an ecotropic viral polypeptide, for example, ecotropic env, which aids entry into cells of the original host species.

Examples of viral polypeptides capable of aiding entry of retroviruses into cells include but are not limited to: MMLV amphotropic env, MMLV ecotropic env, MMLV xenotropic env, vesicular stomatitis virus-g protein (VSV-g), HIV-1 env, Gibbon Ape Leukemia Virus (GALV) env, RD114, FeLV-C, FeLV-B, MLV 10A1 env gene, and variants thereof, including chimeras. Yee et al. (1994) Methods Cell Biol., Pt A:99-112 (VSV-G); U.S. Pat. No. 5,449,614. In some cases, the viral polypeptide is genetically modified to promote expression or enhanced binding to a receptor.

In general, a recombinant virus is produced by introducing a viral DNA or RNA construct into a producer cell. In some cases, the producer cell does not express exogenous genes. In other cases, the producer cell is a "packaging cell" comprising one or more exogenous genes, e.g., genes encoding one or more gag, pol, or env polypeptides and/or one or more retroviral gag, pol, or env polypeptides. The retroviral packaging cell may comprise a gene encoding a viral polypeptide, e.g., VSV-g that aids entry into target cells. In some cases, the packaging cell comprises genes encoding one or more lentiviral proteins, e.g., gag, pol, env, vpr, vpu, vpx, vif, tat, rev, or nef. In some cases, the packaging cell comprises genes encoding adenovirus proteins such as E1A or E1B or other adenoviral proteins. For example, proteins supplied by packaging cells may be retrovirus-derived proteins such as gag, pol, and env; lentivirus-derived proteins such as gag, pol, env, vpr, vpu, vpx, vif, tat, rev, and nef; and adenovirus-derived proteins such as E1A and E1B. In many examples, the packaging cells supply proteins derived from a virus that differs from the virus from which the viral vector derives.

Packaging cell lines include but are not limited to any easily-transfectable cell line. Packaging cell lines can be based on 293T cells, NIH3T3, COS or HeLa cell lines. Packaging cells are often used to package virus vector plasmids deficient in at least one gene encoding a protein required for virus packaging. Any cells that can supply a protein or polypeptide lacking from the proteins encoded by such virus vector plasmid may be used as packaging cells. Examples of packaging cell lines include but are not limited to: Platinum-E (Plat-E), Platinum-A (Plat-A), BOSC 23 (ATCC CRL 11554) and Bing (ATCC CRL 11270). Morita et al. (2000) Gene Therapy 7(12):1063-1066; Onishi et al. (1996) Experimental Hematology, 24:324-329; U.S. Pat. No. 6,995,009. Commercial packaging lines are also useful, e.g., Ampho-Pak 293 cell line, Eco-Pak 2-293 cell line, RetroPack PT67 cell line, and Retro-X Universal Packaging System (all available from Clontech).

The retroviral construct may comprise: a promoter, a multi-cloning site, and/or a resistance gene. The retroviral construct may also comprise a packaging signal (e.g., a packaging signal derived from the MFG vector; a psi packaging signal). Examples of some retroviral constructs known in the art include but are not limited to: pMX, pBabeX or derivatives thereof. Onishi et al. (1996) Experimental Hematology, 24:324-329. In some cases, the retroviral construct is a self-inactivating lentiviral vector (SIN) vector. Miyoshi et al. (1998) J. Virol. 72(10):8150-8157. In some cases, the retroviral construct is LL-CG, LS-CG, CL-CG, CS-CG, CLG or MFG. Miyoshi et al. (1998) J. Virol. 72(10):8150-8157; Onishi et al. (1996) Experimental Hematology, 24:324-329; Riviere et al. (1995) Proc. Natl. Acad. Sci., 92:6733-6737. Virus vector plasmids (or constructs), include: pMXs, pMxs-IB, pMXs-puro, pMXs-neo (pMXs-IB is a vector carrying the blasticidin-resistant gene instead of the puromycin-resistant gene of pMXs-puro) Kimatura et al. (2003) Experimental Hematology 31: 1007-1014; MFG Riviere et al. (1995) Proc. Natl. Acad. Sci., 92:6733-6737; pBabePuro; Morgenstern et al. (1990) Nucleic Acids Research 18:3587-3596; LL-CG, CL-CG, CS-CG, CLG Miyoshi et al. (1998) J. Vir. 72:8150-8157 and the like as the retrovirus system, and pAdexl Kanegae et al. (1995) Nucleic Acids Research 23:3816-3821 and the like as the adenovirus system. In exemplary embodiments, the retroviral construct comprises blasticidin (e.g., pMXs-IB), puromycin (e.g., pMXs-puro, pBabePuro); or neomycin (e.g., pMXs-neo). Morgenstern et al. (1990) Nucleic Acids Research 18:3587-3596.

Methods of producing recombinant viruses from packaging cells and their uses are well established; see, e.g., U.S. Pat. Nos. 5,834,256; 6,910,434; 5,591,624; 5,817,491; 7,070,994; and 6,995,009. Many methods begin with the introduction of a viral construct into a packaging cell line. The viral construct may be introduced into a host fibroblast by any method known in the art, including but not limited to: a calcium phosphate method, a lipofection method (e.g., Felgner et al. (1987) Proc. Natl. Acad. Sci. 84:7413-7417), an electroporation method, microinjection, Fugene transfection, nucleofection and the like, and any method described herein.

Methods of producing recombinant viruses from packaging cells and their uses are well established; see, e.g., U.S. Pat. Nos. 5,834,256; 6,910,434; 5,591,624; 5,817,491; 7,070,994; and 6,995,009. Many methods begin with the introduction of a viral construct into a packaging cell line. The viral construct may be introduced into a host fibroblast by any method known in the art, including but not limited to: a calcium phosphate method, a lipofection method (e.g., Felgner et al. (1987) Proc. Natl. Acad. Sci. 84:7413-7417), an electroporation method, microinjection, Fugene transfection, nucleofection and the like, and any method described herein.

The isolated nucleic acids can be introduced into a cell using a variety of well-known techniques, such as non-viral based transfection of the cell. In an exemplary aspect a construct is incorporated into a vector and introduced into a cell. Introduction into the cell may be performed by any non-viral based transfection method known in the art, such as, but not limited to, electroporation, calcium phosphate mediated transfer, nucleofection, sonoporation, heat shock, magnetofection, liposome mediated transfer, microinjection, microprojectile mediated transfer (nanoparticles), cationic polymer mediated transfer (DEAE-dextran, polyethylenimine, polyethylene glycol (PEG) and the like, or cell fusion. Other methods of transfection include transfection reagents such as Lipofectamine™, Dojindo Hilymax™, Fugene™, jetPEI™, Effectene™, and DreamFect™.

Expression Control Sequences

The nucleotide sequence includes at least one expression control sequence. An expression control sequence is a nucleotide sequence that directs transcription of the nucleic acid of interest. Non-limiting examples of expression control sequences include promoters, enhancers, and silencers. According to the invention, the nucleotide sequence includes a promoter. An expression control sequence can be native or heterologous. A native expression sequence is derived from the same organism, species or strain as the nucleic acid being expressed. A heterologous expression control sequence is derived from a different organism, species or stain as the nucleic acid sequence being expressed.

In some embodiments, the promoter is constitutively active, such as CMV, HSV1-TK, SV40, EF-1α, β-actin, phosphoglycerol kinase (PGK). In other embodiments, the promoter is inducible, such as those containing Tet-operator elements.

Non-limiting examples of suitable eukaryotic promoters (promoters functional in a eukaryotic cell) include CMV, CMV immediate early, HSV thymidine kinase, early and late SV40, long terminal repeats (LTRs) from retrovirus, and mouse metallothionein-I.

Specific gene expression in a selected cell type can be achieved at the level of targeted transcription through the use of tissue/cell-specific promoters and/or enhancers. Cell-specific promoters are one of the primary means through which specialized cellular functions are limited to a particular differentiated cell type. The ability of these promoters to direct transcription of associated genes is regulated, in part, by the intracellular concentrations and activities of transcription factors in the specific type of cell. Due to their specificity, cell-specific promoters may provide a level of transcription of a transgene that is acceptable to cellular metabolism, thus avoiding the exhaustion of protein synthesis materials and over-accumulation of transgene products that may be toxic to transfected cells. Cell-specific promoters can also be advantageous as they may reduce the chance of activating host cell defense machinery and are typically less sensitive to cytokine-induced promoter inactivation than other promoters, for example, viral promoters.

In some embodiments, the promoter is a tissue specific promoter, for example a neuron specific promoter. Non-limiting examples of suitable neuron specific promoters include synapsin I (SYN), calcium/calmodulin-dependent protein kinase II, tubulin alpha I, neuron-specific enolase and platelet-derived growth factor beta chain promoters and hybrid promoters by fusing cytomegalovirus enhancer (E) to those neuron-specific promoters.

In some embodiments, promoters that are capable of conferring cardiac specific expression will be used. Non-limiting examples of suitable cardiac specific promoters include desmin (Des), alpha-myosin heavy chain (α-MHC), myosin light chain 2 (MLC-2), cardiac troponin T (cTnT) and cardiac troponin C (cTnC).

In some embodiments, an enhancer is operably linked to the promoter. An enhancer comprises at least one nucleotide sequence capable of increasing transcriptional activity of the transgene. Generally, enhancers and promoters act to increase and/or activate transcription once bound by appropriate molecules such as transcription factors. For various enhancers which may be used, transcription factor binding sites may be known or identified by one of ordinary skill using methods known in the art, for example by DNA footprinting, gel mobility shift assays, and the like. The factors may also be predicted on the basis of known consensus sequence motifs.

Detectable Marker

In some embodiments, the isolated nucleic acid sequences (e.g., GEDIs) of the present disclosure have been optimized to brightly signal (e.g., fluoresce) only when the $Ca^{2+}$ level within the cell is equivalent, or substantially equivalent, to extracellular $Ca^{2+}$ levels, which make them ideal sensors of cell death.

In many, if not all, cell types, the ability to maintain a gradient of $Ca^{2+}$ between the inside and outside of the cell is essential to life. Once the $Ca^{2+}$ level inside the cell is equivalent, or nearly equivalent, to that outside the cell that cell can be classified as dead.

The fluorescent detectable signals provide extremely robust fluorescence signal, facilitating the use of the GEDIs disclosed herein for use on even the most basic microscopy setup.

According to the invention, the isolated nucleic acid sequence comprises detectable markers that facilitates identification or selection of cells that are undergoing a specific cellular process (e.g., cell death or cell stress). The marker may be detectable by itself (e.g., radioisotope or fluorescent label) or, in the case of an enzymatic marker, may catalyze chemical alteration of a substrate compound or composition which is detectable. The markers can be suitable for small scale detection or more suitable for high-throughput screening. As such, suitable markers include, but are not limited to, radioisotopes, fluorochromes, chemiluminescent compounds, dyes, and proteins, including enzymes. A response that is simply detected generally comprises a response whose existence merely is confirmed, whereas a response that is quantified generally comprises a response having a quantifiable (e.g., numerically reportable) value such as an intensity, polarization, and/or other property. In luminescence or fluorescence assays, the detectable response may be generated directly using a luminophore or fluorophore associated with an assay component actually involved in binding, or indirectly using a luminophore or fluorophore associated with another (e.g., reporter or indicator component).

According to the invention, the isolated nucleic acid sequence comprises two detectable markers (e.g., a first detectable marker and a second detectable marker). Two detectable markers allows for pseudo-ratiometric standardization and assessing cellular morphology. In some embodiments, the first detectable marker, the second detectable marker, or both comprise a fluorescent label. In some embodiments, the first detectable maker, the second detectable marker, or both comprise a luminescent probe, for example, aequorin or luciferase. According to the invention, when two (or more) detectable markers are used, the first detectable marker and the second detectable marker are distinguishable from each other.

Examples of suitable fluorescent detectable markers include, but are not limited to, genes encoding fluorescent proteins, e.g., green fluorescent protein (GFP), enhanced-GFP (EGFP), Ds-Red (DsRed: *Discosoma* sp. red fluorescent protein (RFP); Bevis et al. (2002) Nat. Biotechnol. 20(11):83-87), yellow fluorescent protein (e.g., Venus), mCherry, mApple, blue-green emission tyrosine-derived chromophore (GEM), cyanofluorescent protein (e.g., Turquoise), variants thereof, and the like.

In one embodiment the isolated nucleic acid sequence comprises RGEDI-P2A-X, wherein X represents a detectable fluorescent marker that is distinguishable from the red death indicator RGEDI, for example, EGFP. In another embodiment, the isolated nucleic acid sequence comprises GGEDI-P2A-X, wherein X represents a detectable fluorescent marker that is distinguishable from the green death indicator GGEDI, for example, mApple. In another embodiment, the isolated nucleic acid sequence comprises RGEDI-P2A-X, wherein X represents a detectable fluorescent marker that is distinguishable from the red death indicator RGEDI, for example, TagBFP2. In yet another embodiment, the isolated nucleic acid sequence comprises GEM-GEDI-P2A-X, wherein X represents a detectable fluorescent marker that is distinguishable from the green to blue death indicator GEM-GEDI, for example, Venus. In still another embodiment the isolated nucleic acid sequence comprises low-affinity red fluorescent (LAR)-GEDI-P2A-X, wherein X represents a detectable fluorescent marker that is distinguishable from the red death indicator LAR-GEDI, for example, EGFP.

In some embodiments, at least one detectable marker can be proteins conferring resistance to a selection agent, e.g., a neomycin resistance gene, a puromycin resistance gene, a blasticidin resistance gene, and the like.

Calcium Binding Motif

The isolated nucleic acid GEDIs of the present invention comprise a modified calcium binding motif.

In one embodiment, the modified calcium binding motif comprises an optimized single fluorescent protein $Ca^{2+}$ indicator, GCaMP. GCaMP consists of circularly permuted green fluorescent protein (cpGFP) tagged to Calmodulin and an M13 protein. Calmodulin (*i.e.*, CALcium MODULated proteIN) is found in the cytoplasm and acts as an intermediary protein

that senses $Ca^{2+}$ levels and relays signals to various calcium-sensitive enzymes, ion channels and other proteins. When $Ca^{2+}$ concentration increases in a cell, the calmodulin component of GCaMP binds to $Ca^{2+}$ and causes a conformational change in the cpGFP that result in increased fluorescence.

GCaMP variants can be constructed by any method known in the field, including, for example, by PCR of the coding region using mutagenic primers and/or site-directed mutagenesis at selected positions. Non-limiting examples of GCaMP variants include GCaMP1, GCaMP2, GCaMP3, GCaMP4, GCaMP5, and GCaMP6. In some embodiments, one or more GCaMP variants are expressly excluded.

A modified calcium binding motif may comprise a nucleotide sequence having at least about 60%, at least about 65%, at least about 70%, at least about 75%, at least about 80%, at least about 85%, at least about 90%, at least about 95%, at least about 99%, or 100% nucleotide sequence identity of SEQ ID NO. 1 or SEQ ID NO. 2.

In some embodiments, the modified calcium binding motif comprises a nucleotide sequence having at least about 90% nucleotide sequence identity to a nucleotide region of SEQ ID NO. 1, wherein the nucleotide region comprises about 100, about 200, about 300, about 400, about 500, about 600, about 700, about 800, about 900, about 1000, about 1100, about 1200, or about 1300 contiguous nucleotides of SEQ ID NO. 1.

A modified calcium binding motif may comprise a nucleotide sequence having at least about 90% nucleotide sequence identity to a nucleotide region of SEQ ID NO. 2, wherein the nucleotide region comprises about 100, about 200, about 300, about 400, about 500, about 600, about 700, about 800, about 900, about 1000, about 1100, or about 1200 contiguous nucleotides of SEQ ID NO. 2.

A modified calcium binding motif may comprise a polypeptide sequence having at least about 60%, at least about 65%, at least about 70%, at least about 75%, at least about 80%, at least about 85%, at least about 90%, at least about 95%, at least about 99%, or 100% nucleotide sequence identity of SEQ ID NO. 3 or SEQ ID NO. 4.

Any calcium binding motif domains are contemplated by the present disclosure. In some embodiments, the calcium binding motif comprises at least 1, at least 2, at least 3, at least 4, at least 5, or more EF-hand motifs. In some embodiments, the modified calcium binding motif comprises Troponin C (TnC).

The calcium binding motifs of the present disclosure can be modified to provide an optimized signal (e.g., fluoresce) only when the $Ca^{2+}$ level within the cell is equivalent, or substantially equivalent, to extracellular $Ca^{2+}$ levels, which make them ideal sensors of cell death.

In many, if not all, cell types, the ability to maintain a gradient of $Ca^{2+}$ between the inside and outside of the cell is essential to life. Once the $Ca^{2+}$ level inside the cell is equivalent, or nearly equivalent, to that outside the cell that cell can be classified as dead.

In some embodiments, the calcium binding motifs can be modified to reduce its $Ca^{2+}$ binding affinity. In some embodiments that binding affinity has been reduced by at least a factor of about 5, about 10, about 20, about 50, about 100, about 200, about 300, about 400, about 500, about 600, about 700, about 800, about 900, about 1000, about 1500, about 2000, about 2500, or more compared to naturally occurring motif.

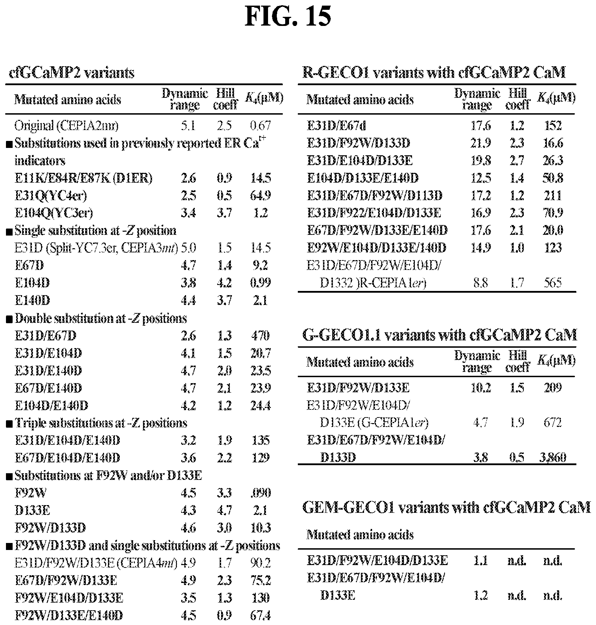

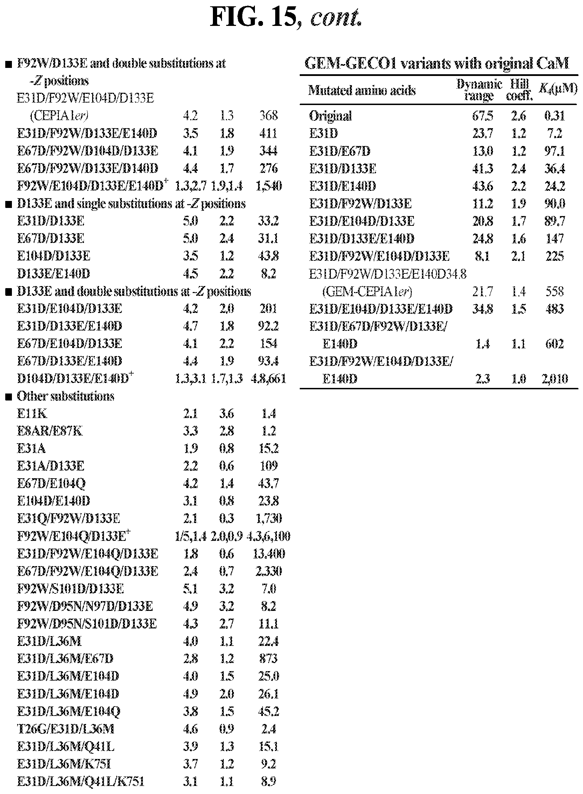

$Ca^{2+}$ binding affinity of calmodulin may be reduced by modifying (e.g., addition, deletion or substitution) of at least one amino acid of SEQ ID NO. 5. $Ca^{2+}$ binding affinity of calmodulin may be reduced by modifying (e.g., addition, deletion or substitution) more than 1, 2, 3, 4, 5, 6, 7, 8, 9, or 10 amino acids of SEQ ID NO. 5. the substitution of SEQ ID NO. 5 comprises at least one of E11K, T26G, E31A, E31Q, E31D, L36M, Q41L, E67D, K751, E84R, E87K, F92W, S101D, E104Q, E104D, D133E, E140D, or any combination thereof. The modified calcium binding motif of SEQ ID NO. 5 may comprises any one of the amino acid substitutions or combination of substitutions of FIG. 15. According to the invention, the substitution of SEQ ID NO. 5 comprises at least one of E31D, F92W, E104D and D133E The modified calcium binding motif of SEQ ID NO. 5 may comprise an amino acid substitution or combination of substitutions selected from the group consisting of E31D, E31D/F92W/D133E, E31D/F92W/E104D/D133E, E31D/E67D/F92W/E104D/D133E, E31D/F92W/E104D/D133E. In one embodiment, the modified calcium binding motif comprises substitutions of E31D/F92W/E104D/D133E of SEQ ID NO. 5.

As used herein dissociation constant ($K_d$) refers to the binding affinity of components to one another, for example $Ca^{2+}$ to a calcium binding protein, such as calmodulin or troponin C. In some embodiments, the isolated nucleic acid sequence has a $K_d$ greater than 1 μM, greater than 10 μM, greater than 100 μM, greater than 200 μM, greater than 250 μM greater than 300 μM, greater than 350 μM, greater than 400 μM, greater than 450 μM greater than 500 μM, greater than 550 μM, greater than 600 μM, greater than 650 μM greater than 700 μM, greater than 750 μM, greater than 800 μM, greater than 850 μM, greater than 900 μM, greater than 950 μM, or more.

Isolated nucleic acid sequences may comprise a localization sequence, for example, a nuclear localization sequence, an endoplasmic reticulum localization sequence, a mitochondrial localization sequence, or any combination thereof. Isolated nucleic acid sequences may comprise a localization sequence (e.g., a nuclear localization sequence), for example, a nucleic acid sequence according to SEQ ID NO. 11. A localization sequence (e.g., a nuclear localization sequence) may be added to the 3' of the GEDI (e.g., RGEDI) and/or of the detectable fluorescent marker (e.g., EGFP). A localization sequence (e.g., a nuclear localization sequence) may be added to the 5' of the GEDI (e.g., RGEDI) and/or of the detectable fluorescent marker (e.g., EGFP). In one embodiment, the isolated nucleic acid sequences expressly exclude a localization sequence. According to the invention, the isolated nucleic acid sequence excludes a nuclear localization sequence, an endoplasmic reticulum localization sequence, a mitochondrial localization sequence, or any combination thereof.

Non-limiting examples of suitable GEDI constructs comprise nucleotide sequences having at least about 60%, at least about 65%, at least about 70%, at least about 75%, at least about 80%, at least about 85%, at least about 90%, at least about 95%, at least about 99%, or 100% nucleotide sequence identity of SEQ ID NOs. 6, 7, 8, 9, or 10. SEQ ID NO. 10 has a tandem (3x) TagBFP2 sequence to increase intensity of the BFP fluorescence. SEQ ID NO. 7 (Hsyn1:RGEDI-P2a-TagBFP2) works but, at least in some tests, had a lower efficiency as compared to SEQ ID NO. 10 (Hsyn1:RGEDI-P2a-3xTagBFP2).

*Cells*

The present disclosure also provides isolated cells comprising the nucleic acid sequence and/or vectors of an embodiment described herein. The cell can be, for example, a eukaryotic cell or a prokaryotic cell. The cells can be of any appropriate species, e.g., an animal such as a mammal, for example, a canine, an equine, a feline, or a human cell. Suitable cells include, for example, yeast cells (e.g., *S. cerevisiae* and *Candida*), bacteria cells (e.g., *Neisseria, Streptococcus, Staphylococcus*), and plant cells. Non-limiting examples of human cells include, neurons, cardiac cells, fibroblasts, and red blood cells. In one embodiment, the cell is a neural cell, a cardiac cell, a muscle cell, a cancer cell, or a gamete.

As used herein "neuron" refers to an animal cell consisting of a cell body and one or more protrusions that extrude from the cell body, i.e., an axon or neurite, and several dendrites, and examples of the neuron may include sensory neurons, motor neurons (motoneuron), and interneurons. In addition, the neuron may include neurons constituting a central nervous system, a brain, brain stem, spinal cord and synaptic regions of the central nervous system and peripheral nervous systems, neurosustentacular cells, glia, and Schwann cells. Neurons may be derived from stem cells, including, for example, embryonic stem cells, induced pluripotent stem cells, or neural stem cells.

Cardiac cells refer to any cell present in the heart that provides a cardiac function, such as heart contraction or blood supply, or otherwise serves to maintain the structure of the heart. Cardiac cells as used herein encompass cells that exist in the epicardium, myocardium or endocardium of the heart. Cardiac cells also include, for example, cardiac muscle cells or cardiomyocytes, and cells of the cardiac vasculatures, such as cells of a coronary artery or vein. Other non-limiting examples of cardiac cells include epithelial cells, endothelial cells, fibroblasts, cardiac stem or progenitor cells, cardiac conducting cells and cardiac pacemaking cells that constitute the cardiac muscle, blood vessels and cardiac cell supporting structure. Cardiac cells may be derived from stem cells, including, for example, embryonic stem cells or induced pluripotent stem cells.

*Animals*

Also provided herein are transgenic animals that include the isolated nucleic acid GEDIs disclosed herein. "Animal" refers to non-human animals, including, mammals, amphibians and birds. Specifically, examples include bovines, felines, guinea pigs, horses, mice, non-human primates, ovines, porcines, rabbits, rats, sheep, zebrafish and the like. As used herein, transgenic animal refers to any animal in which one or more of the cells of the animal contain a heterologous nucleic acid. Methods for making transgenic animals have been described, for example, in Wagner et al. (1981) Proc. Nat. Acad. Sci. USA, 78:5016-5020; Stewart et al. (1982) Science, 217:1046-1048; Constantini et al. (1981) Nature, 294:92-94; Lacy et al. (1983) Cell, 34:343-358; McKnight et al. (1983) Cell, 34:335-341; Brinstar et al. (1983) Nature, 306:332-336; Palmiter et al. (1982) Nature, 300:611-615; Palmiter et al. (1982) Cell, 29:701-710; and Palmiter et al. (1983) Science, 222:809-814. Such methods are also described in U.S. Pat. Nos. 6,175,057; 6,180,849; and 6,133,502.

By way of example, the transgenic animal can be created by introducing a nucleic acid into, for example, an embryonic stem cell, an unfertilized egg, a fertilized egg, a spermatozoon or a germinal cell containing a primordial germinal cell thereof, preferably in the embryogenic stage in the development of a non-human mammal (more preferably in the single-cell or fertilized cell stage and generally before the 8-cell phase). The nucleic acid can be introduced by known means, including, for example, the calcium phosphate method, the electric pulse method, the lipofection method, the agglutination method, the microinjection method, the particle gun method, the DEAE-dextran method and other such method. Optionally, the nucleic acid is introduced into a somatic cell, a living organ, a tissue cell or other cell by gene transformation methods. Cells including the nucleic acid may be fused with the above-described germinal cell by a commonly known cell fusion method to create a transgenic animal.

For embryonic stem (ES) cells, an ES cell line may be employed, or embryonic cells may be obtained freshly from a host, e.g., mouse, rat, guinea pig, and the like. Such cells are grown on an appropriate fibroblast-feeder layer or grown in the presence of appropriate growth factors, such as leukemia inhibiting factor (LIF). When ES cells have been transformed, they may be used to produce transgenic animals. After transformation, the cells are plated onto a feeder layer in an appropriate medium. Cells containing the construct may be detected by employing a selective medium. After sufficient time for colonies to grow, they are picked and analyzed for the occurrence of homologous recombination or integration of the nucleic acid. Those colonies that are positive may then be used for embryo manipulation and blastocyst injection. Blastocysts are obtained from 4 to 6 week old superovulated females. The ES cells are trypsinized, and the modified cells are injected into the blastocoel of the blastocyst. After injection, the blastocysts are returned to each uterine horn of pseudopregnant females. Females are then allowed to go to term and the resulting litters screened for mutant cells having the construct. By providing for a different phenotype of the blastocyst and the ES cells, chimeric progeny can be readily detected. The chimeric animals are screened for the presence of the nucleic acid, and males and females having the modification are mated to produce homozygous progeny transgenic animals.

Induced pluripotent stem cells (iPSCs) may also be employed. The term "induced pluripotent stem cells" shall be given its ordinary meaning and shall also refer to differentiated mammalian somatic cells (e.g., adult somatic cells, such as skin) that have been reprogrammed to exhibit at least one characteristic of pluripotency. See, for example, Takahashi et al. (2007) Cell 131(5):861-872, Kim et al. (2011) Proc. Natl. Acad. Sci. 108(19): 7838-7843, Sell (2013) Stem Cells Handbook.

In some aspects the disclosure provides, organotypic slice cultures comprising cells having the isolated nucleic acid sequence or vector as described herein. As used herein, the term "organotypic slice culture" refers to sections or explants of tissue which are maintained in culture (Kang (2016) Sci. Rep. 6:28798 | DOI: 10.1038/srep28798; Seil (1979) Review in Neuroscience 4:105-177; Gahwiler (1981) J Neurosci Meth 4:329-342; Gahwiler (1984) Neuroscience 11:751-760, Gahwiler (1988) Trends Neurosci 11:484-490; Stoppini et al. (1991) J Neurosci Methods 37:173-182). A skilled artisan can readily employ art known organotypic slice culture methods for use in the present invention.

An organotypic brain slice culture refers to sections or explants of brain tissue and can employ sections of whole brain tissue or explants obtained from specific regions of the brain. Any region can be used to generate an organotypic brain slice culture. However, a preferred source of the organotypic brain slice culture is explants obtained from specific regions of the brain, preferably the hippocampus or cortex region.

*Methods of Use*

Also provided are methods for monitoring calcium flux in a cell comprising isolated nucleic acid as described herein comprising: measuring the signal from the first detectable marker to generate a first signal intensity; measuring the signal from the second detectable marker to generate a second signal intensity; and comparing the first signal intensity to the second signal intensity to generate a signal intensity ratio.

Also provided are methods for monitoring cell death in a cell comprising isolated nucleic acid described herein comprising: measuring the signal from the first detectable marker to generate a first signal intensity; measuring the signal from the second detectable marker to generate a second signal intensity; comparing the first signal intensity to the second signal intensity to generate a signal intensity ratio; and determining whether the cell is dead or alive based on the signal intensity ratio.

Also provided are methods of screening for an agent that is capable of causing an intracellular calcium concentration fluctuation in a cell comprising: contacting a cell comprising the isolated nucleic acid described herein with the agent; measuring the signal from the first detectable marker to generate a first signal intensity; measuring the signal from the second detectable marker to generate a second signal intensity; comparing the first signal intensity to the second signal intensity to generate a signal intensity ratio; and determining whether the agent is capable causing an intracellular calcium concentration fluctuation based the signal intensity ratio.

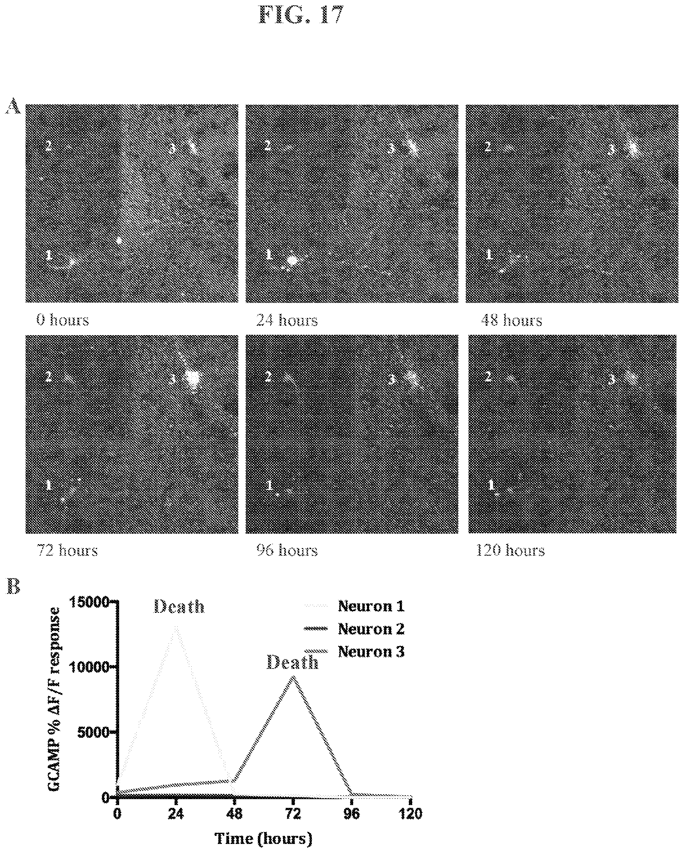

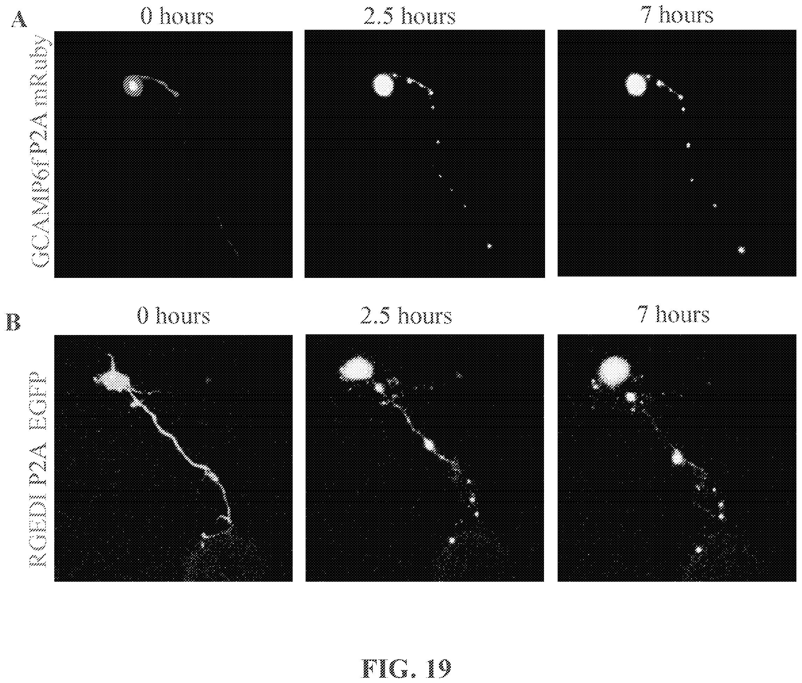

In some embodiments, the methods for monitoring cell death, monitoring calcium flux, and/or screening for an agents in a cell comprise use of optical means by, for example, a microscope, a spectrophotometer, a fluorimeter, a cooled charge-coupled device (CCD) imager, a fluorescence activated cell sorter (FACS). Non-limiting examples of microscopes useful for the methods described herein include inverted microscopes, confocal microscopes, including spinning disk confocal and scanning confocal, and the like. In one embodiment, automated time-lapse confocal microscopy is used to detect $Ca^{2+}$ transients, for example, during neurodegeneration. In some embodiments, the method uses epifluorescence microscopy, for example, as shown in FIG. 17 and FIG. 19.

In some embodiments, the method is a high-throughput method, automated methods, or both. In some embodiments, the method is an automated time lapse as described in Arrasate et al. (2014) Nature 431:805-810. In some embodiments the methods utilize in vivo imaging techniques.

In some embodiments, a signal intensity of greater than 0.001, 0.01, 0.02, 0.03, 0.04, 0.05, 0.06, 0.07, 0.08, 0.09, 0.10, 0.20, 0.25, 0.30, 0.35, 0.40, 0.45, 0.50, 0.55, 0.60, 0.65, 0.70, 0.75, 0.80, 0.85, 0.90, 0.95, 1.0, or greater indicates the cell is dead. In one embodiment, a signal intensity ratio of greater than 0.05 indicates the cell is dead. In some embodiments, it is contemplated that different imaging setups will likely report different relative GEDI signal intensity ratios, necessitating a control experiment to empirically determine the appropriate GEDI signal intensity ratio. In some embodiments, a signal intensity ratio is empirically determined by, for example, inducing cell death and finding dead cells and live cells based on morphological markers or using a complimentary acut live/dead marker on GEDI expressing cells (e.g., TUNEL stain or PhiPhiLux) and using signals from those populations of cells to generate a signal intensity ratio. For example, the signal intensity ratio can be determined by taking the average signal from about 10 dead cells less the average signal from about 10 live cells. In some embodiments the signal intensity ratio is determined using the formula: *signal intensity ratio* = ([(*GEDI dead ratio*) - (*GEDI live ratio*)] * 0.25) + [*avg. GEDI live*].

In some embodiments, cell death is apoptosis, necrosis, autophagy, necroptosis, or any combination thereof.

In some embodiments, the first intensity signal detects cell morphology. In other embodiments, the second intensity signal detects cell morphology.

In some embodiments, the first intensity signal detects calcium binding to the calcium binding motif. In other embodiments, the second intensity signal detects calcium binding to the calcium binding motif.

In some embodiments, cell death is determined when $Ca^{2+}$ levels inside the cell are equivalent to $Ca^{2+}$ levels outside the cell.

*Kits*

Also provided herein are kits comprising one or more of the isolated nucleic acids or vectors disclosed herein, any of the cells or organotypic slice cultures disclosed herein, or one or more of the non-human animals disclosed herein. In some embodiments, the kit further comprises instructions for monitoring calcium flux, monitoring cell death, and/or screening for an agent that is capable of causing an intracellular calcium concentration fluctuation in a cell. In some embodiments, the kit further comprises instructions for monitoring calcium flux, monitoring cell death, and/or screening for an agent that is capable of causing an intracellular calcium concentration fluctuation in an organotypic slice culture. In some embodiments, the kit further comprises instructions for monitoring calcium flux, monitoring cell death, and/or screening for an agent that is capable of causing an intracellular calcium concentration fluctuation in a non-human animal.

EXAMPLES

Example 1: Genetically Encoded Calcium Indicators (GECIs) for Monitoring Cell Death

Genetically Encoded Calcium Indicators (GECI) have conventionally been used in the field of neuroscience in live imaging to detect $Ca^{2+}$ transients triggered by synaptic input and action potential firing. Surprisingly, through time-lapse imaging over long periods of time and quantitative data analysis, it was discovered that GCaMP could be used in detecting neuronal cell death. When a cell dies, its membrane invariably breaks down, allowing extracellular $Ca^{2+}$ ions to rush into the cell and disrupt the $Ca^{2+}$ buffering and homeostasis within the neuron and that is required for neuronal function. Nevertheless, cellular proteins and debris remain intact and within the membrane shell long after the membrane integrity is lost. It was also discovered that biosensors, such as GFP and GCaMP remain functional within the debris, emitting fluorescence long after the cell is dead. Therefore, it was contemplated that a large increase in GCaMP when a cell dies could be used to distinguish between live and dead cells.

Rat primary cortical neuron cultures were prepared from embryos (embryonic day 16-18) and co-transfected using Lipofectamine2000 (ThermoFisher Scientific, Carlsbad, CA, USA) with 150ng/well of hSyn1:mRuby-P2a-GCaMP (Addgene #50943) and either Htt[ex1]-Q27 or Htt[ex1]-Q97 (known to induce neurodegeneration) in a 96-well format. Robotic microscopy was used to image and track individual neurons once every 24 hours for 6 days as previously described by Arraste et al., (2004) starting at 5 days in vitro (DIV). FIG. 17A. The GCaMP intensity was detected and plotted as a percentage increase from baseline (t=0) (*i.e.*, delta GCaMP/baseline GCaMP). Using the morphology as a readout, the time of death was determined for each neuron. FIG. 17B shows Neuron 1 was determined dead at approximately 24 hours, Neuron 2 remained alive throughout the duration of the time course, and Neuron 3 was determined dead at approximately 72 hours.

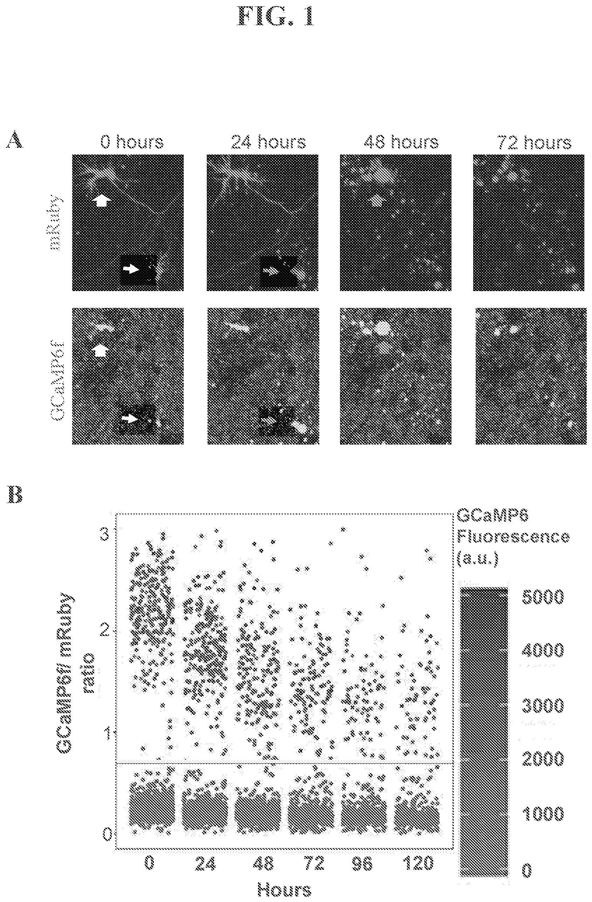

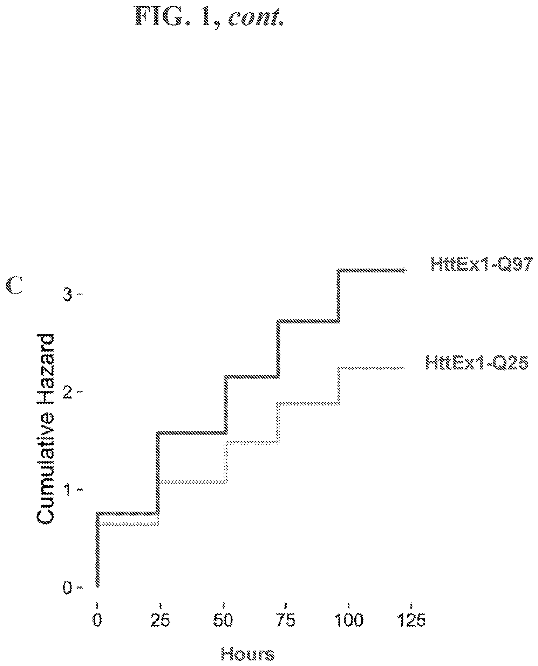

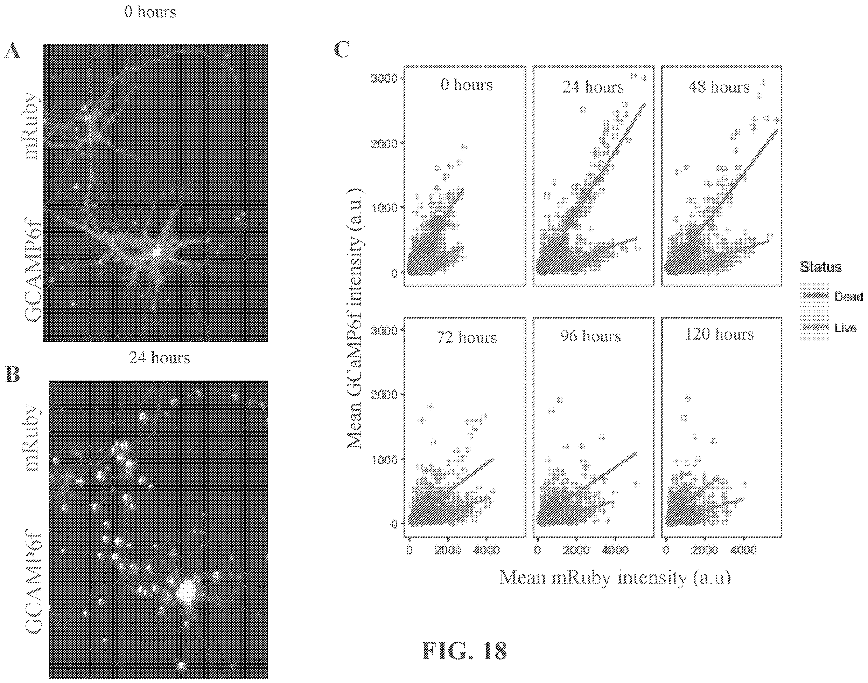

To examine the changes in signal increase as a cell dies, rat primary cortical neurons expressing GCaMP6f, mRuby, and HttEx1-Q97 were examined overtime. As shown in FIG. 1A, as neurons die there is an increase in GCaMP6f expression. Using time lapse imaging, it was observed that 0 out of 94,106 dead rat primary cortical neurons examined regenerated after elevated GEDI signal, indicating elevated GEDI signal is terminal. In addition, the ratio of GCaMP6f/m-Ruby signal from rat primary cortical neurons expressing HttEx1-Q97 and HttEx1-Q25 at 0, 24, 48, 72, 96, and 120 hours post transfection was determined. FIG. 1B, the horizontal line at 0.75 separates dead from live neurons. Finally, data showed that HttEx1-Q97 is more toxic than HttEx1-Q25. FIG. 18A shows a single neuron expressing Htt[ex1]-Q97 that is alive at 0 hrs (5 DIV) and FIG. 18B shows the same neuron after 24 hrs (6 DIV). Similarly, human motor neurons differentiated from induced pluripotent stem cells were transfected with 0.2 μg/well hSyn1:mRuby-P2a-GCaMP and imaged with robotic microscopy every 6 hours up to 12 hours. FIG. 19A shows increase in GCaMP signal in individual human motor neurons.

These bioinformatics data analysis techniques demonstrated that a pseudo-ratiometric GCaMP6f-P2A-mRuby was capable of distinguishing live from dead neurons in culture with a high degree of accuracy.

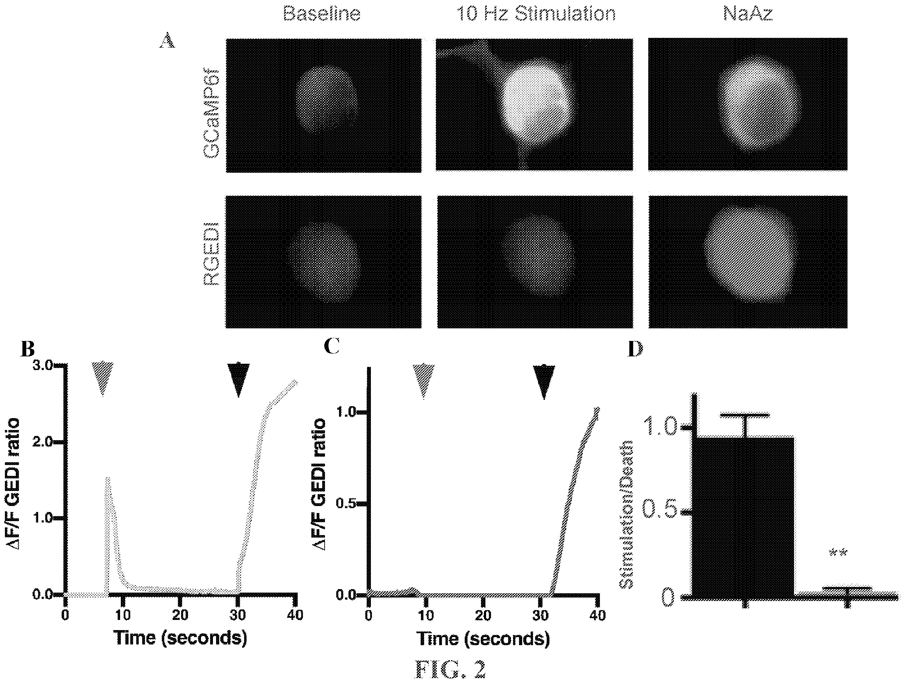

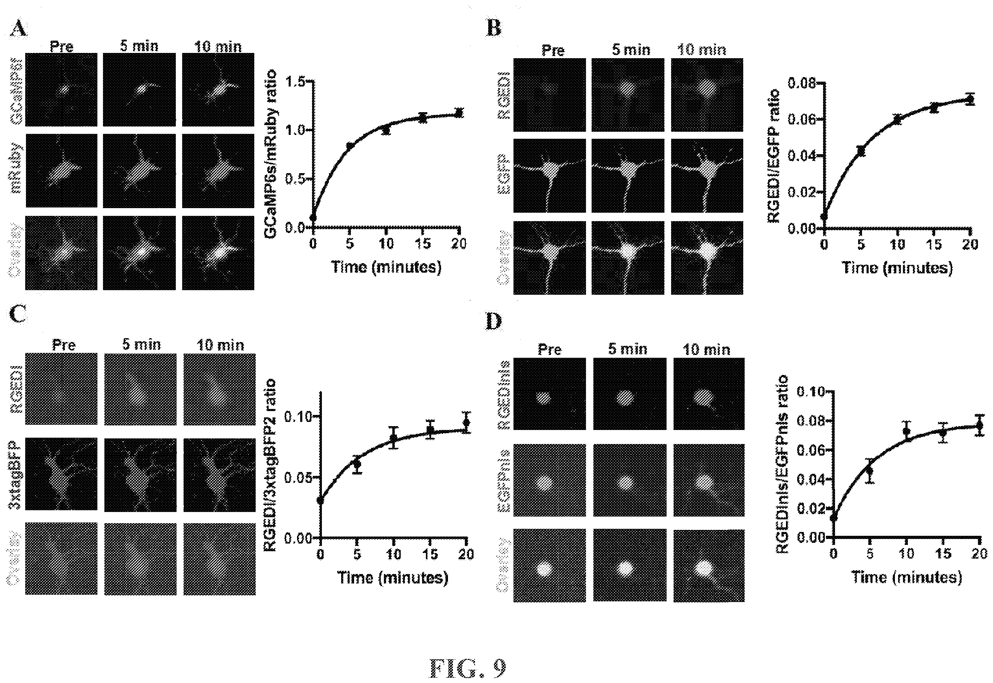

Time lapse imaging was performed on cells having either GCaMP6f or RGEDI to determine specificity of response following exposure to different stimuli. As shown in FIGs. 2A-2D, after 10Hz voltage stimulation no significant change in RGEDI signaling was observed as compared to baseline control. However, after NaAz exposure to induce death a large change in RGEDI signaling was observed. This data demonstrates that RGEDI responds only at death of the neuron and not during voltage stimulation. In addition, GEDI ratio time lapse response to neurons after addition of NaAz showed that the GEDI ratio responds reproducibly with each GEDI variant tested. FIG. 9.

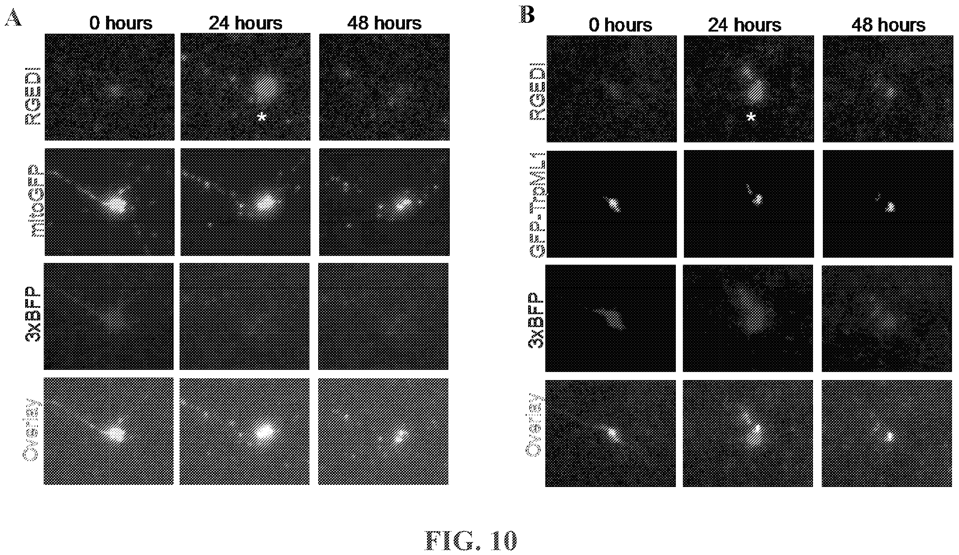

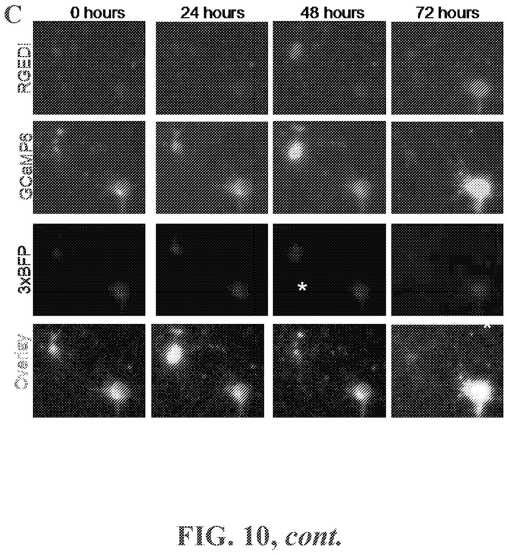

The RGEDI were also analyzed for their ability to be multiplexed with detectors for parallel analysis of multiple cellular observations. Here, RGEDI-P2a-tag3xBFP was co-transfected with an independent green channel biosensor. A mitochondrial marker mitoGFP, a lysosomal marker GFP-TrpML1, and a cytosolic Ca2+ indicator were each recorded in parallel with the GEDI death indicator at 0, 24, 48 and 72 hours post transfection. Time of neuronal death is indicated with a white asterisk. FIG. 10A-C. These data demonstrate that GEDIs can be used in multiplexed analyses and do not impede observations of other cellular reporters.

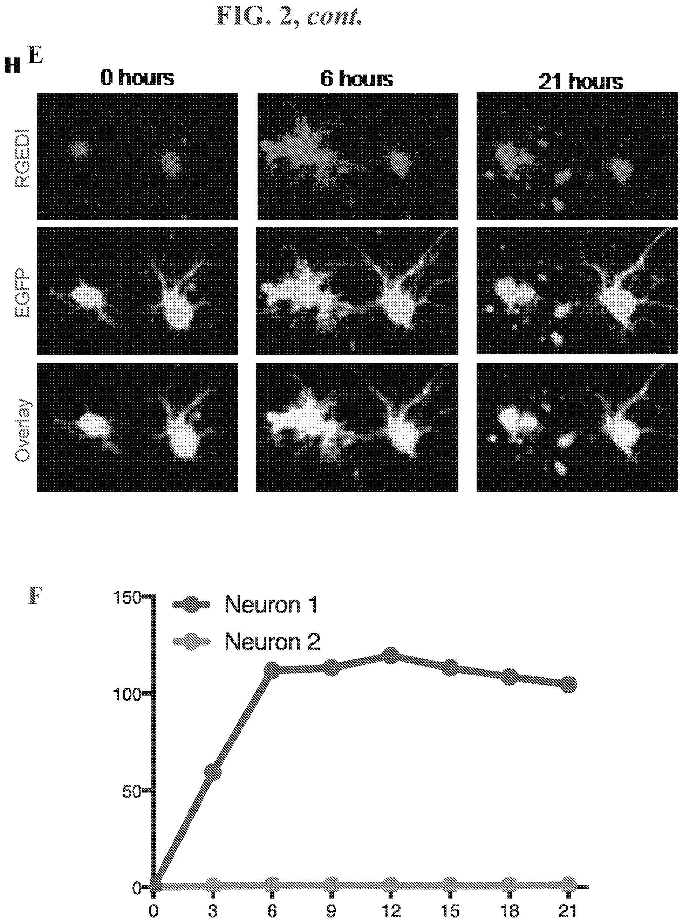

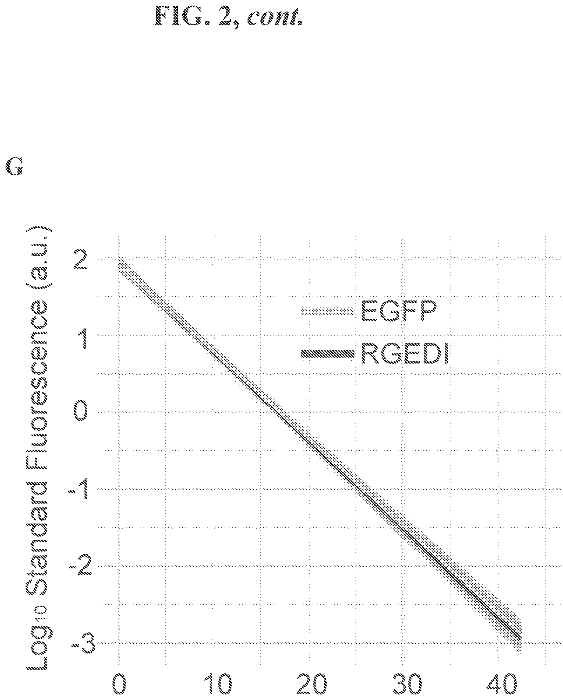

To further analyze the GEDIs, time lapse experiments were performed on rat primary cortical neurons expressing RGEDI-P2a-EGFP. Images were collected of both dying and healthy neurons at 0, 6, and 21 hours post transfection. As seen in FIG. 2E, the RGEDI expression level increases in the dying neuron (left) while the healthy, surviving neuron (right) maintains a baseline level of expression during the course of the experiment. FIG. 2F shows quantification of the $\Delta F/F$ RGEDI/EGFP ratio of neurons from FIG2E from 0 to 21 hours after transfection and show that the ratio increases in the dead neuron but does not in the live neuron. Analysis of decay rates showed that RGEDI and EGFP fluorescence decay at the same rate after death stably for up to 42 hours after death. FIG. 2G. Together this showed for the first time GCaMP could be modified for use as genetically-encoded cell death indicators. GEDIs are an alternative to other commercially available cell death indicators with several advantages. GEDIs are genetically encoded with low toxicity, they are death-type agnostic, provide acute and robust signal, and can have a dual role as a $Ca^{2+}$/neuronal activity indicator.

Example 2: Development of Genetically Encoded Death Indicators

The pseudo-ratiometric GCaMP6f-P2A-mRuby were capable of distinguishing live from dead neurons, but because the GCaMP was engineered to fluoresce with a dynamic range based on physiological levels of $Ca^{2+}$ with a healthy neuron, fluctuations in GCaMP fluorescence blurred the separation between live and dead GCaMP fluorescent neurons. In addition, interpretation of cell death was complicated because these GEDIs were not ratiometric indicators. In addition, because fluorescent debris fades when it dissolves/diffuses, it was difficult to differentiate long dead versus live cells. Thus, it was desirable to generate a construct that could more accurately and/or precisely distinguish a live and health neuron with $Ca^{2+}$ transients and a dead neuron that is beyond the point of recovering its membrane polarization.

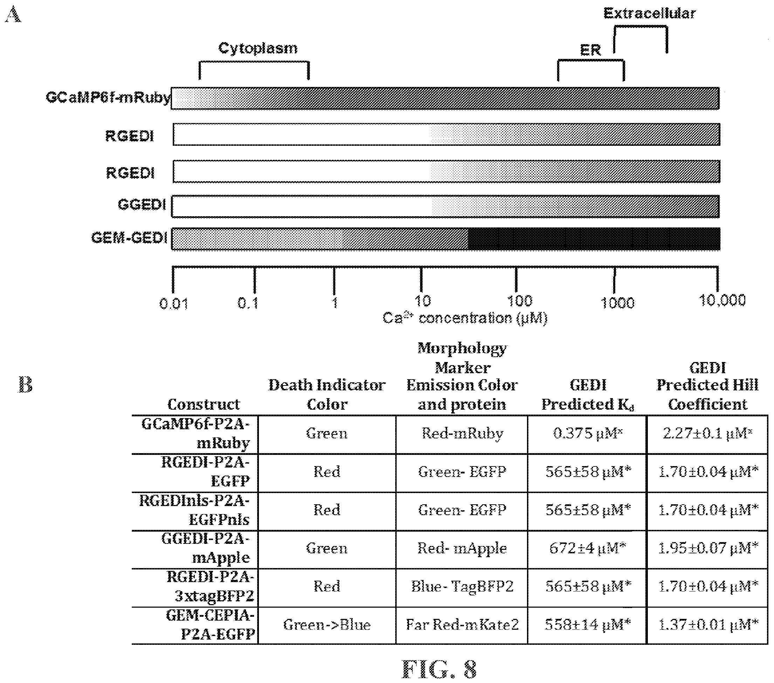

To achieve a higher separation between live and dead neurons and to inhibit the ability of the sensor to increase fluorescence during normal endogenous $Ca^{2+}$ transients within healthy cells, a novel set of genetically encoded indicators were designed. These new constructs were specifically designed, in part, to detect high levels of $Ca^{2+}$ that would not be seen in live, intact neurons. These constructs were designed to remain in the cytosol (i.e., did not contain a subcellular localization signal) and increase only when a cell, for example a neuron, died and extracellular levels of $Ca^{2+}$ are in equilibrium with the cell debris. Additionally, a second genetically encoded fluorophore of a complementary color was attached with a self-cleavable P2A tag for use as a pseudo-ratiometric standardization signal and neuronal morphology marker. FIG. 8A. Color variants were generated, including RGEDI-P2A-EGFP, RGEDI-P2A-mApple, RGEDI-P2A-tagBFP2, RGEDI-P2A-3xtagBFP2 and GEM-GEDI-P2A-EGFP. The in vitro characteristics for each variant were determined along with predicted Hill Coefficients and $K_d$ for $Ca^{2+}$ binding. As shown in FIG. 8B the GEDI variants of the present disclosure all have an average predicted $K_d$ greater than 500 $\mu$M.

Example 3: GEDIs as In Vitro Cell Death Reporters

To examine use of these novel GEDIs as effective reporters of cell death in vitro, hSyn1:RGEDI-P2A-EGFP was expressed in rat cortical primary neurons. Rat primary cortical neuron cultures were prepared and robotic microscopy was used to image and track individual neurons once every 24 hours for six days as described above and previously described (Arraste et al., 2004) beginning at 5 days in vitro (DIV).

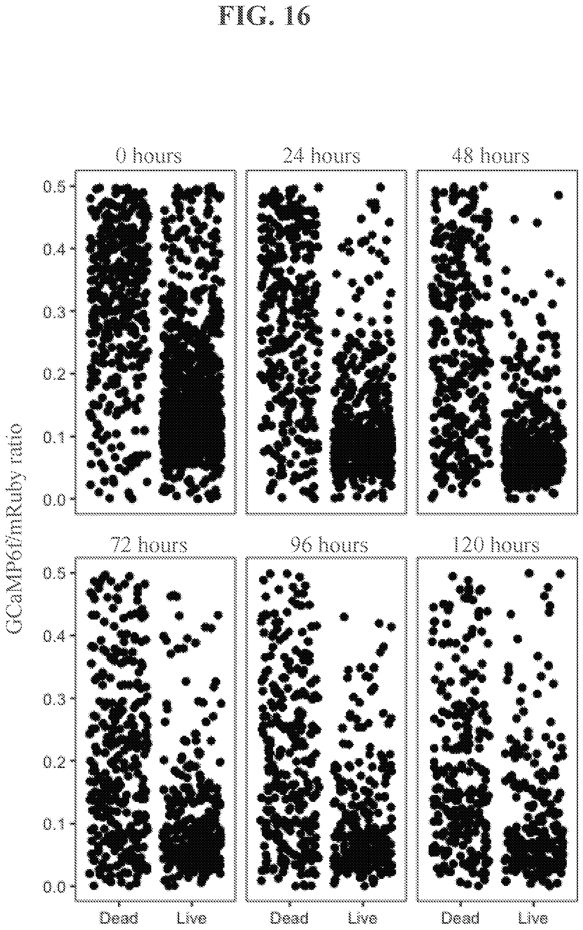

Individual neurons were tracked over the course of 120 hours. As shown in FIG. 4B, the GEDIs were better able to distinguish dead and live signals as compared to the earlier GCaMP6f-P2A-mRuby construct in FIG. 16.

To use the constructs as cell death indicators, a "live" and "dead" signal ratio was first determined that could then be interpolated to other samples. While this method was developed using live in vitro neurons, it is contemplated that the same or similar method can be used to define quantity of cell death in live (e.g., in vitro or in vivo), single time-point or time lapse, or fixed cells of all types.

Briefly, to establish the "live" GEDI ratio, media was removed from cells, washed twice, and replaced with $Ca^{2+}$-

free media with addition of 1mM EGTA. Ten representative cells expressing RGEDI-P2a-EGFP were imaged separately with a RFP and GFP filter set. Once the imaging parameters were set, the same parameters were used for all further measurements. The GEDI signal was calculated for all cells using the following formula:

$$GEDI_{signal} = \text{average of [mean RFP fluorescence intensity within cell}$$
$$\text{body]/[mean GFP fluorescence intensity within the cell}$$
$$\text{body] for all cells measured}$$

Next, to rapidly induce cell death, 2% NaN$_3$ was added to the well for 10 minutes. The same cells expressing RGEDI-P2a-EGFP with an RFP and GFP filter set were imaged using the same parameters as set above. The GEDI signals were then calculated. This GEDI signal value should not be significantly different than the first signal ratio recorded, due to the use of Ca-free medium with EGTA. Use this GEDI signal as the "live" GEDI ratio.

To establish the "dead" GEDI ratio, in a separate well, ten representative cells expressing the RGEDI-P2a-EGFP were imaged separately with an RFP and GFP filter set using the same imaging parameters as above. The GEDI signal was calculated for those ten cells. To rapidly induce cell death, 2% NaN$_3$ was then added to the well for 10 minutes. The ten cells were again imaged with an RFP and GFP filter set with same imaging parameters. The GEDI signals for the ten cells were determined. This was used as the "dead" GEDI ratio.

The following formula was then used to compute the GEDI threshold:

$$GEDI_{threshold} = 0.25 \text{ x ([“live” GEDI ratio ] + [“dead” GEDI ratio])}$$

A cell with a measured GEDI signal above this threshold using the same imaging and technical conditions was considered a dead cell. A cell with a measured GEDI signal below this threshold using the same imaging and technical conditions was considered a live cell. For single time point and time-lapse imaging of live cells, a "live" and "dead" signal ratio was established at onset of imaging and that ratio and same imaging parameters was used for all subsequent time points. For time-lapse imaging over more than 24 hours, single cell death must be propagated as GEDI "dead" signal will fade after 24 hours (*i.e.*, report as "alive" based on signal intensity). For fixed cells, the same protocol was followed for establishing a "live" and "dead" signal ratio as described for live cells, but cells were fixed at 10 minute time point after addition of NaN$_3$. After fixation, the "live" and "dead" GEDI signals were determined

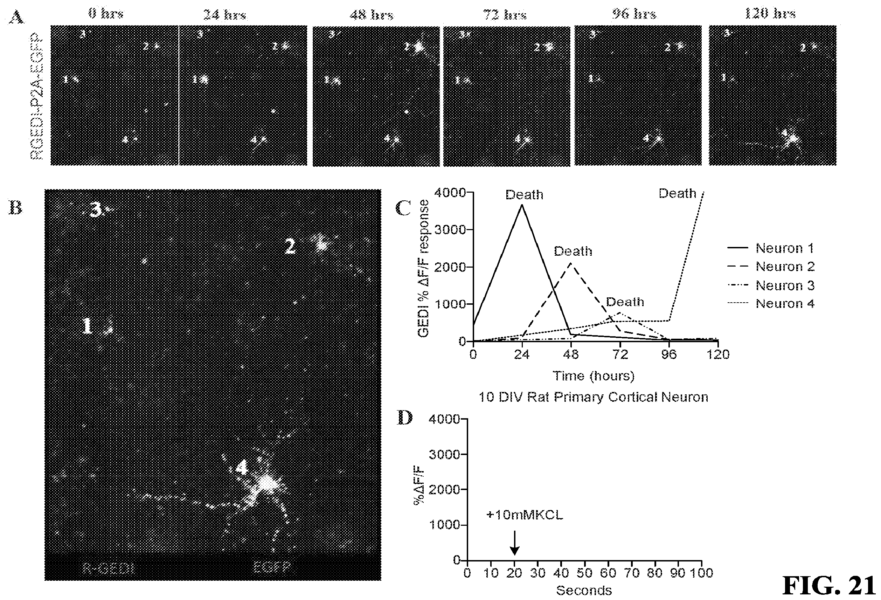

FIG. 21A depicts representative images of individual neurons over the time course. The RGEDI-P2A-EGFP exhibited $\Delta F/F0$ responses to increased intracellular Ca$^{2+}$ levels. Next, the RGEDI intensity was plotted as a percentage increase from baseline (t=0) (ie delta RGEDI/baseline RGEDI). Using the morphology as a readout, the time of death was determined for each neuron. As shown in FIG. 21C, Neurons 1, 2, 3, and 4 were determined to be dead at approximately 24 hours, 48 hours, 72 hours, and 120 hours, respectively. On the other hand, rat primary neurons exposed to KCl, which induces membrane depolarization and rapid Ca$^{2+}$ transients, failed to excite RGEDI-P2A-EGFP. FIG. 21D. This demonstrates that the GEDIs, having decreased binding affinity to Ca$^{2+}$, limit fluorescence above basal state unless the cell membrane becomes permeable to levels of Ca$^{2+}$ beyond which a neuron can recover.

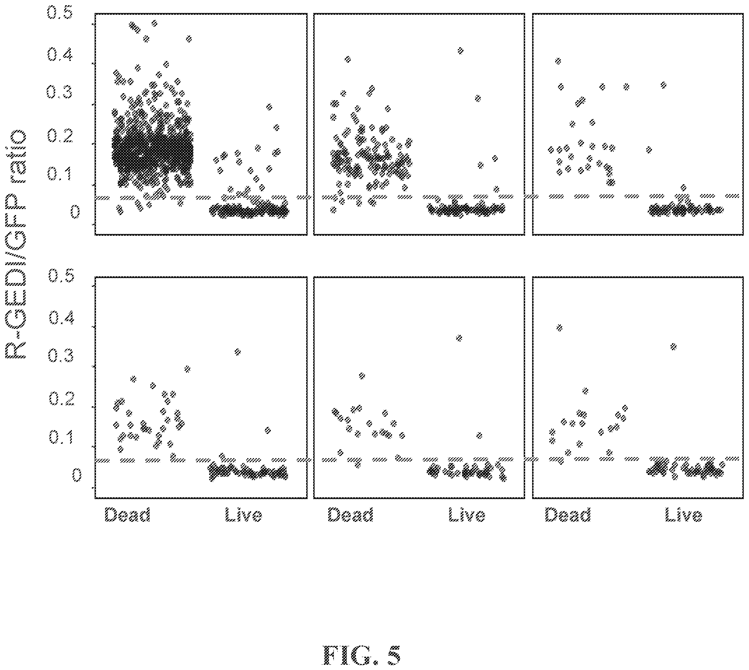

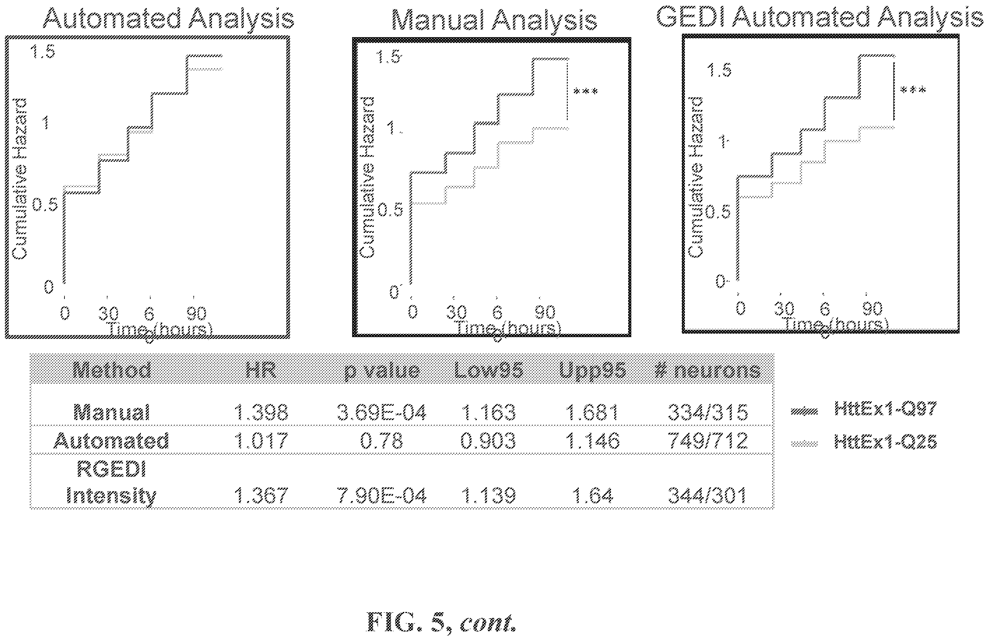

Next, the intensity of RGEDI for individual neurons expressing the amino-terminal exon 1 fragment of huntingtin (HttEx1) containing polyQ$^{97}$was plotted on the y axis in each of 6 consecutive time points with the EGFP intensity on the X axis. HttEx1 containing polyQ$^{97}$ resulted in polyQ-expansion-dependent, neuron-specific cell death at various time points during longitudinal tracking of single neurons. FIG. 5 shows RGEDI was able to detect live versus dead rat primary cortical neurons. RGEDI was able to better distinguish live and dead signals as compared to GCaMP6f-P2A-mRuby (FIG. 16 and FIG. 4).

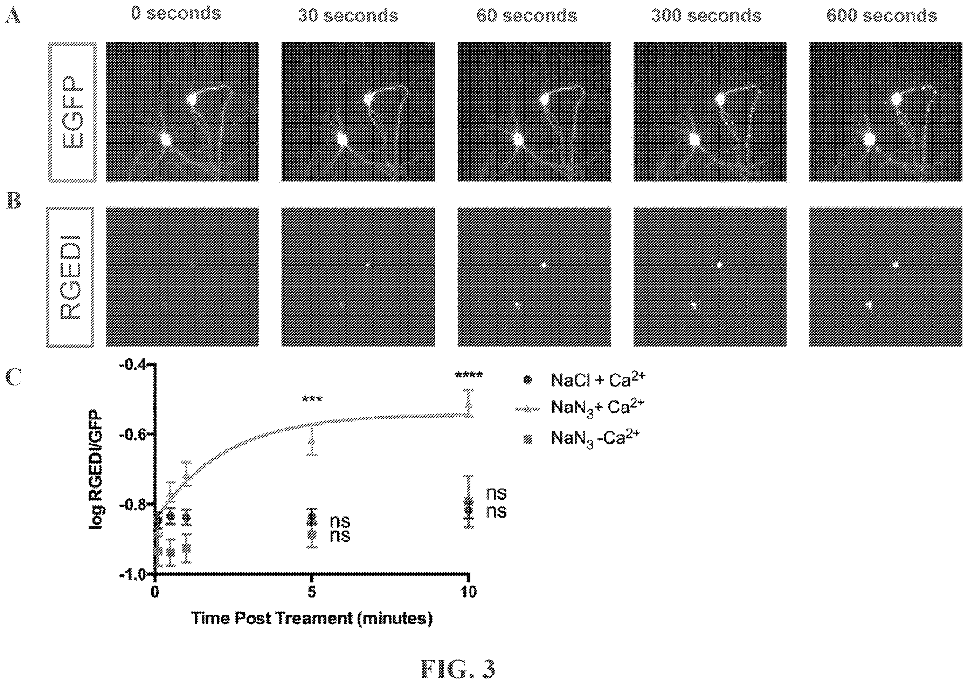

Rat primary neurons were transfected with hsyn1:RGEDI-P2A-EGFP as described above and imaged at DIV10. RGEDI/EGFP ratio significantly increased after addition of 2% NaN$_3$ to neurons in neural basal media (NBM) with physiological Ca2+ levels (~2mM Ca$^{2+}$)( NaN$_3$ + Ca$^{2+}$) compared to neurons with addition of saline (NaCl + Ca$^{2+}$) which does not induce death. A non-linear regression was fit to the NaN$_3$ + Ca$^{2+}$ time course and a half-life of RGEDI/EGFP signal increase was determined to be 5 minutes and 50 seconds. Neurons washed 3x and incubated in Ca2+ free PBS prior to exposure to NaN3 (NaN$_3$ - Ca$^{2+}$) did not show a significant increase in RGEDI/EGFP ratio. ANOVA Tukey comparison to 0 minutes of each condition, *** p<0.001, **** p<0.0001. These data suggest RGEDI signal increase acutely during neuronal death and requires extracellular Ca$^{2+}$. FIG. 3.

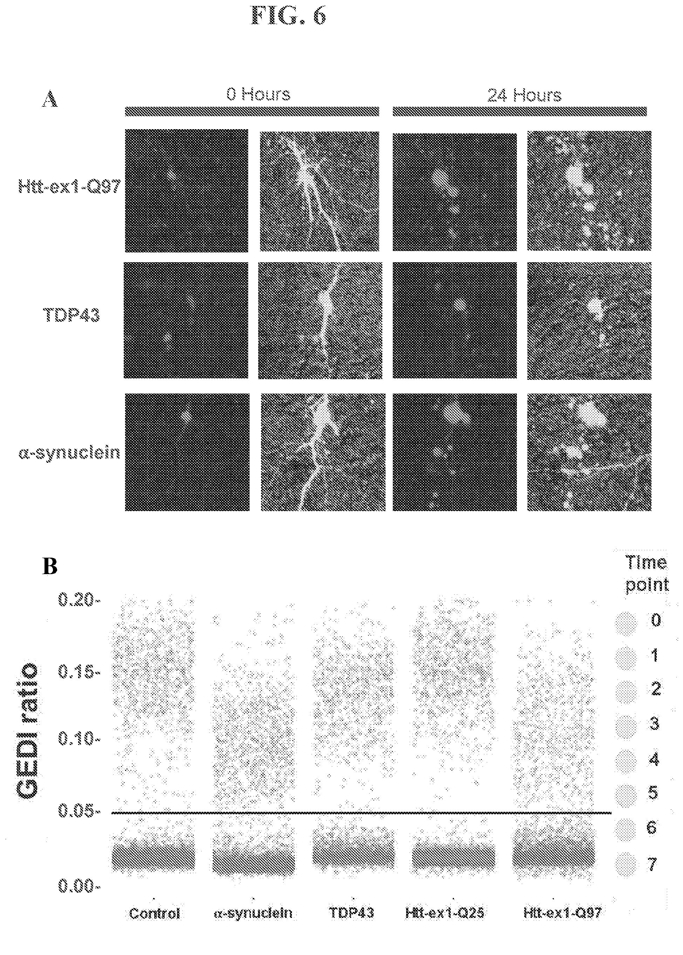

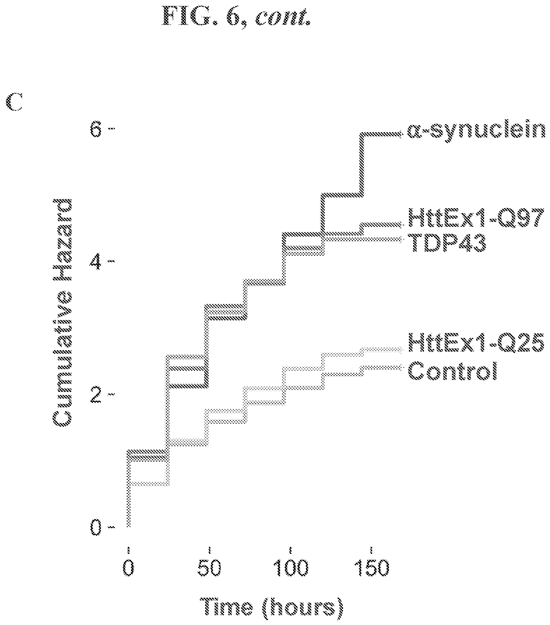

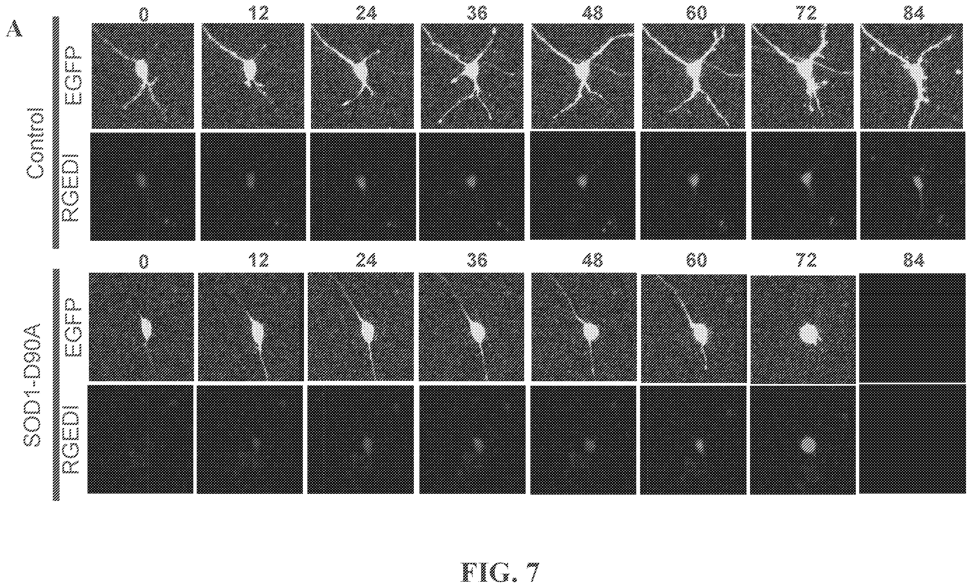

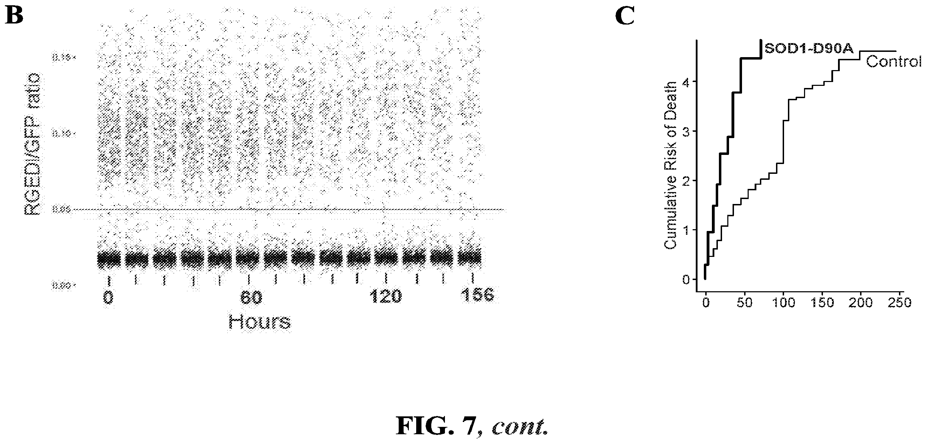

To examine GEDI activity in other neurodegenerative diseases, neurons were transfected with either HttEx1-Q97, TDP43, or $\alpha$-synuclein. All showed an increase in RGEDI signal upon death. FIG. 6A. In addition, observing GEDI ratios derived from neurons expressing HttEx1-Q97, HttEx1-Q25, TDP43, $\alpha$-synuclein compared to control neurons in high throughput using automated imaging demonstrated that while a GEDI threshold set at 0.5 dividing dead and live neurons is often sufficient, it may be necessary to generate a GEDI threshold (i.e., signal intensity ratio) for different studies, for example, studies of different diseases. FIG. 6C shows an automated cumulative hazard plot derived from data in FIG. 6B showing HttEx1-Q97, TDP43, and α-synuclein each have increased toxicity over time compared to control as previously reported in the literature, indicating GEDI can be used on multiple types of neurodegenerative diseases. In addition, FIGs. 7A-C demonstrate that GEDI can be used to study risk of death for certain genetic mutations as compared to wild type controls. Studies were performed on an amyotrophic lateral sclerosis (ALS) model having a copper/zinc superoxide dismutase (SOD1) mutation (D90A-SOD1). It is contemplated that GEDIs can be equally deployed as cell death indicators for other disease models as well.

Example 4: GEDIs as In Vivo and In Tissue Cell Death Reporters

The process of neurodegeneration is often influenced by surrounding tissue, which limits the ability of researchers to study neurodegeneration in vitro. Yet the ability to image cell death in vivo has been hindered by the ability to visualize when cell death occurs in vivo or in tissue, such as organotypic slice culture. In particular, conventional methods do not provide a nontoxic label that is stable over the long time course involved in neurodegeneration. Therefore, it was determined whether GEDIs could serve as reliable cell death reporters in vivo and in tissue.

Briefly, zebrafish embryos were co-injected at 1-cell stage with mnx1:Gal4, UAS:GCaMP6f, and UAS:Nitroreductase-mCherry. Embryos were grown at 28.5 °C until 48 hours post fertilization (hpf), anaesthetized in tricaine, embedded in low-melting point agarose, and then imaged on a spinning disk confocal microscope. Images were acquired of the same motor neurons within the zebrafish at 0, 24 hours, and 48 hours after MTZ or DMSO was added to the media. Intensity of GCaMP6f was plotted as the change in fluorescence intensity from time point 0.

In addition, a hippocampal slice culture was generated from neonatal CB7BL/6 mouse embryos using a Siskiyou tissue slicer to generate 400 $\mu$m thick slices, cultured using the interface method, and maintained at 34°C. Slices were then co-transfected using biolistic transfection by coating gold nanoparticles with RGEDI-P2a-EGFP and htt ex1 97Q, and imaged using a spinning disk confocal microscope. Ten $\mu$m Z slices were taken and collapsed into Max projection Z stacks.

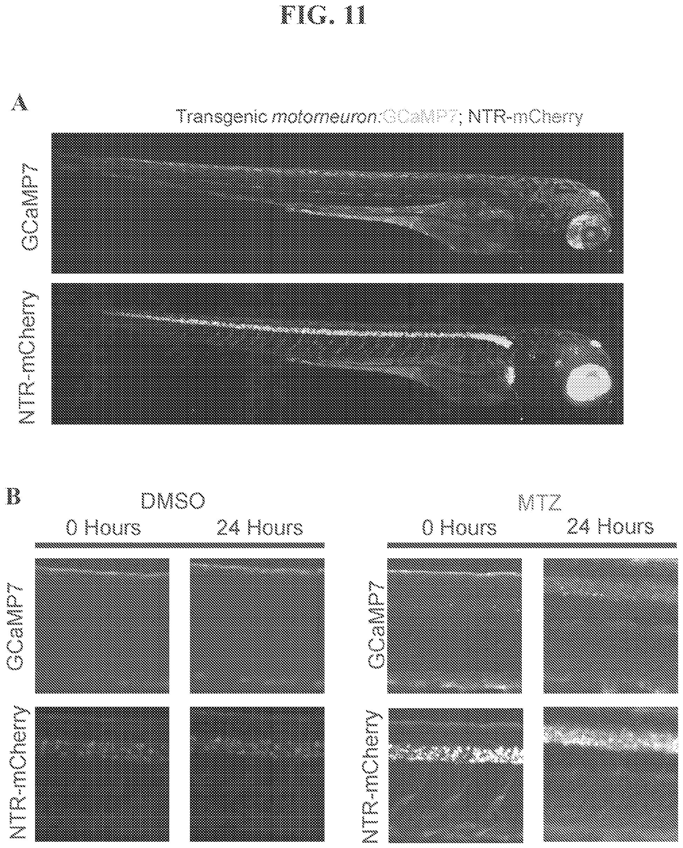

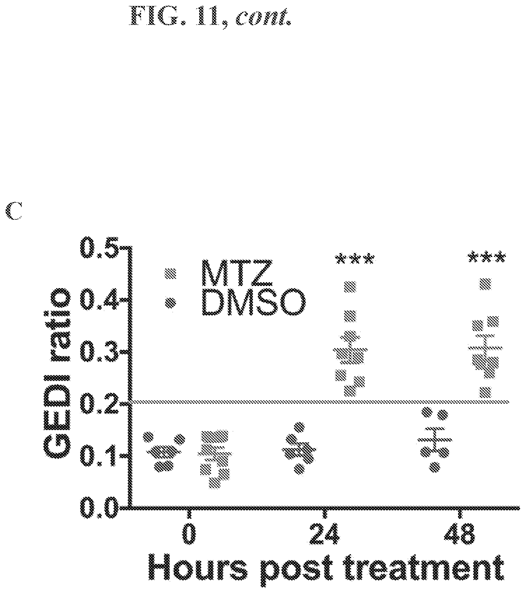

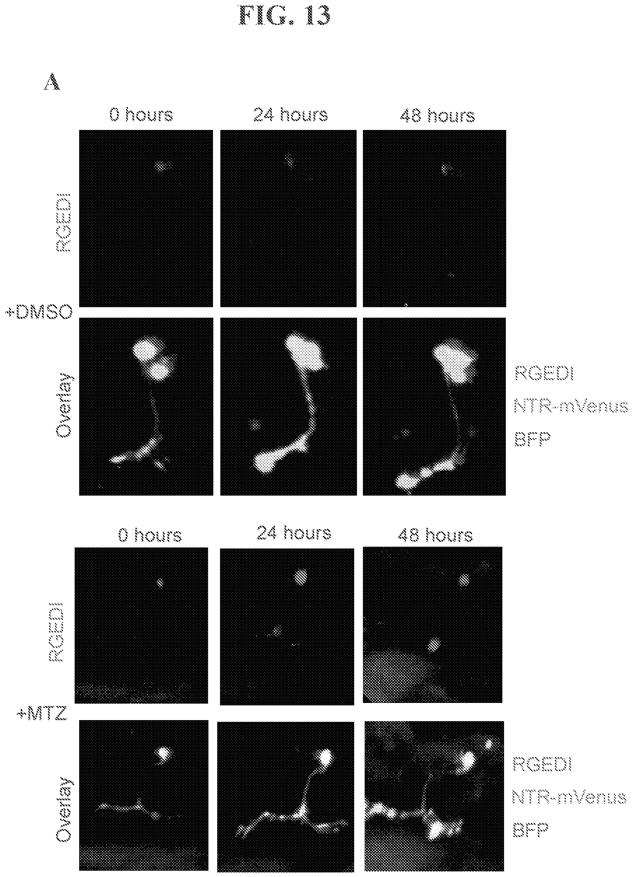

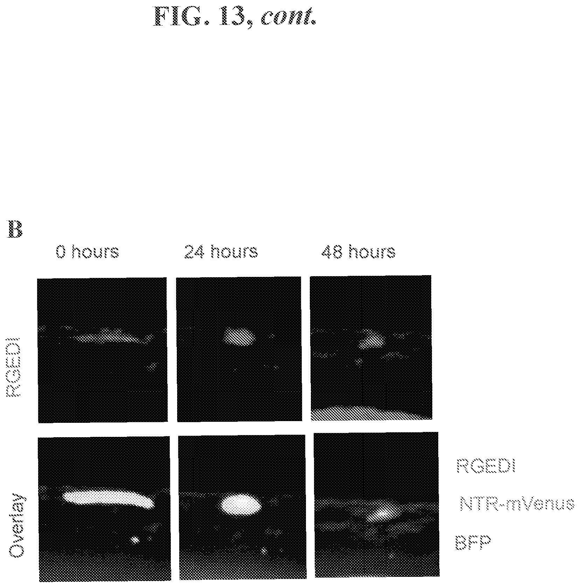

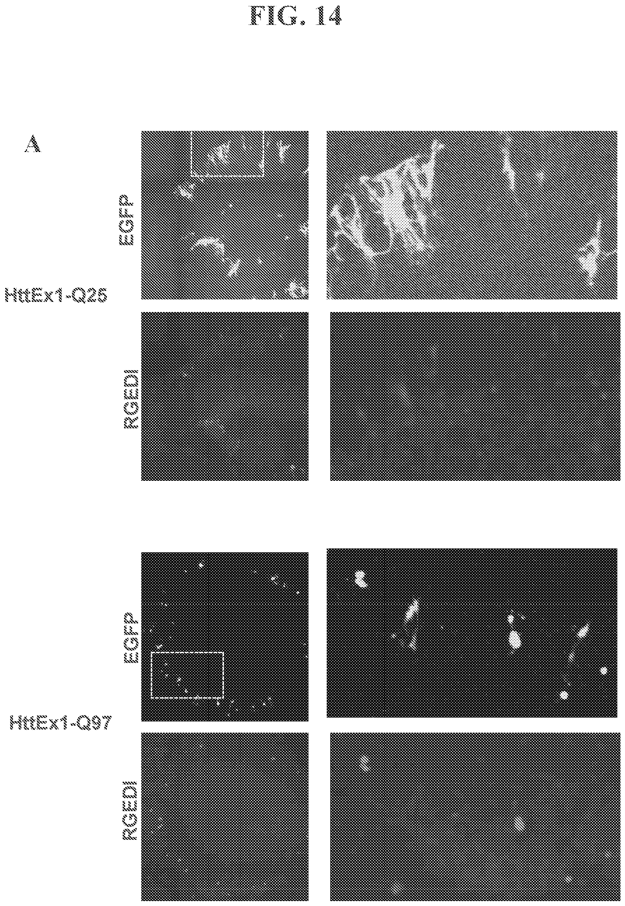

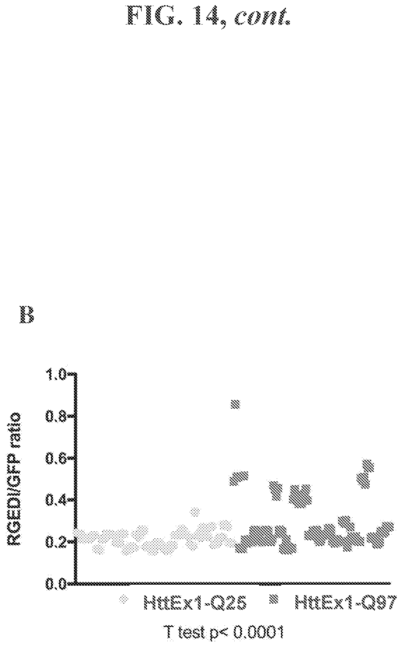

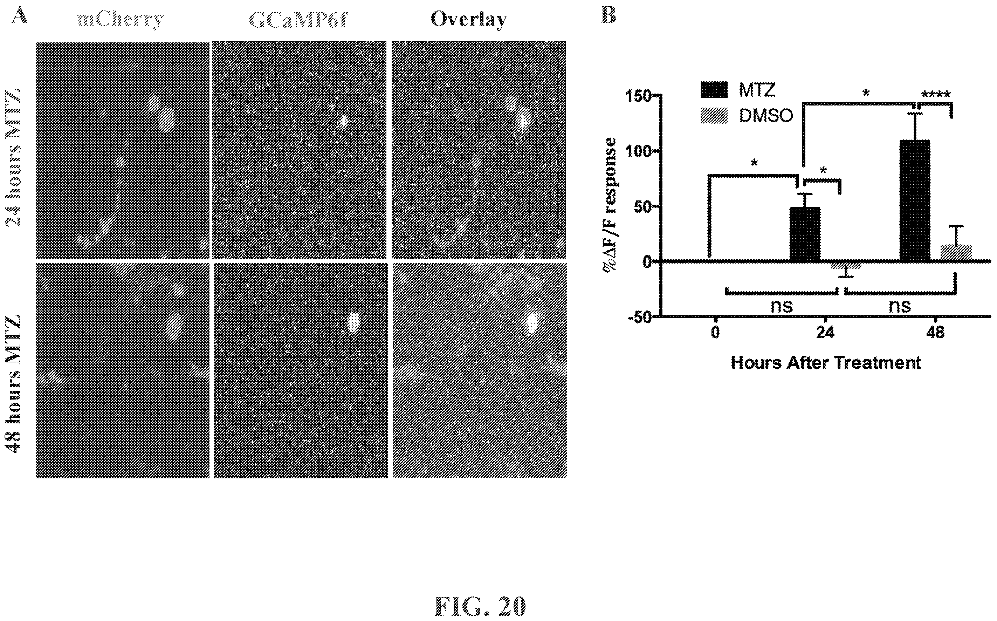

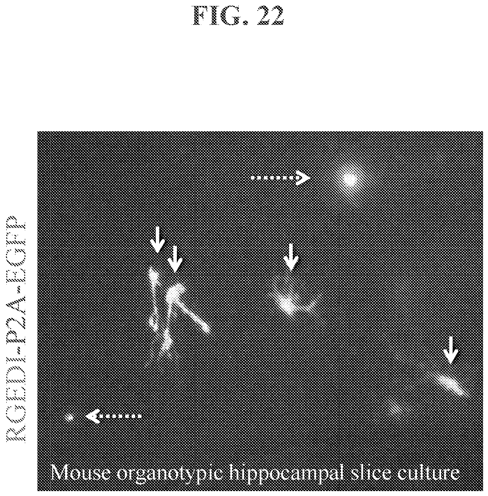

By expressing GCaMP in motor neurons of zebrafish larvae that are anaesthetized in tricaine which diminishes $Ca^{2+}$ transients in neurons (FIG. 11A) and time lapse imaging after target neuronal ablation using induced nitroreductase of those motor neurons, it was demonstrated that GCaMP signal also increases after cell death in vivo. As shown in FIGs. 20A, 11B and 11C, zebrafish larvae GCaMP expression increased at 24 and 48 hours after addition of metronidazole (MTZ) to activate cell ablation, which coincided with a loss of cellular morphology. The GCaMP-P2A-mCherry zebrafish also exhibited %$\Delta$F/F responses to MTZ treatment that were substantially augmented over responses to the DMSO control. RGEDI-P2a-3XtagBFP was also co-expressed in zebrafish neurons with NTR-mVenus and signal increased in neurons exposed to MTZ but not those exposed to DMSO as shown in FIG 13A and 13B. The GEDI reporter was also tested in skeletal muscle fiber. FIG. 13C. In addition, expression of RGEDI-P2A-EGFP was tested in an organotypic slice culture and was shown to be useful for determining neuronal cell death. FIGs. 14A, 14B, and 22.

FIG. 13B contains representative a single zebrafish skeletal muscle fiber co-expressing RGEDI-P2a-3xtagBFP and NTR-mVenus at 0, 24, and 48 hours showing RGEDI signal increases during skeletal muscle fiber death.

These data indicate that these novel GEDI variants are capable for use in vivo and in tissue.

Example 6: GEDIs in Combination with Robotic Microscopy

Next it was examined whether GEDIs were useful in combination with robotic microscopy - the use of automated microscopes to do high-throughput time-lapse imaging on live cells. Robotic microscopy is capable of generating high-throughput time-lapse data, yet detection of cell death had proven difficult to automate accurately. Briefly, automated images were acquired from neurons expressing RGEDI-P2A-EGFP.

Here, using a threshold (dotted line = 0.05) in the ratio of RGEDI/EGFP, neurons were classified as dead or alive for GEDI automated analysis. Briefly, ray primary cortical neurons expressing htt ex1 25Q (normal) or htt ex1 97Q (Huntington's disease model) were induced to undergo cell death. A comparison of survival analysis on the same data set using cumulative hazard ratio for neurons in which death was called after loss of automated segmentation ("Automated Analysis"), death classified using manual curation according to morphology signal ("Manual Analysis"), or death was classified as RGEDI/EGFP signal > 0.05 ("GEDI Automated Analysis"). As shown in FIG. 5B, and summarized in FIG. 5C, use of robotic microscopy with RGEDI-P2A-EGFP, followed by fully automated analysis resulted in comparable hazard ratios and statistical analysis to the same data set manually analyzed. Similarly significant p values and hazard ratios were obtained by manual analysis and GEDI automated analysis, which automated analysis failed to detect a significant difference in survival.

It takes a trained technician approximately 15 hours of labor to analyze a standard 96 well plate for cell death. Surprisingly, the automated-GEDI analysis appeared to out-perform manual curation of data. Manually curated data that was reexamined after looking at GEDI signal is consistently found to be misclassified. (dashed boxes, FIG. 4). Thus, GEDI saves time and money, with improved accuracy. Through manual curation analysis, robotic microscopy with single cell tracking already provides 100-1000 fold increased sensitivity over high-throughput screening systems based on single snapshots. Arrasate M. et al., (2005) Proc Natl Acad Sci U S A., 102(10):3840-5. This demonstrates the ability for GEDIs to increase the accuracy of robotic microscopy even further.

Drug screening has been used to identify toxic compounds. However, the ability to image cell death accurately in high throughput screens has been hindered by the ability to visualize precisely when cell death occurs.

Glutamate is an amino acid and one of the most plentiful neurotransmitters in the brain. It is present only in small amounts, and it is the primary excitatory neurotransmitter. Too much glutamate can lead to excitotoxicity which can destroy neurons. Some diseases are believed to be linked to glutamate sensitivities, for example, amyotrophic lateral sclerosis (ALS).

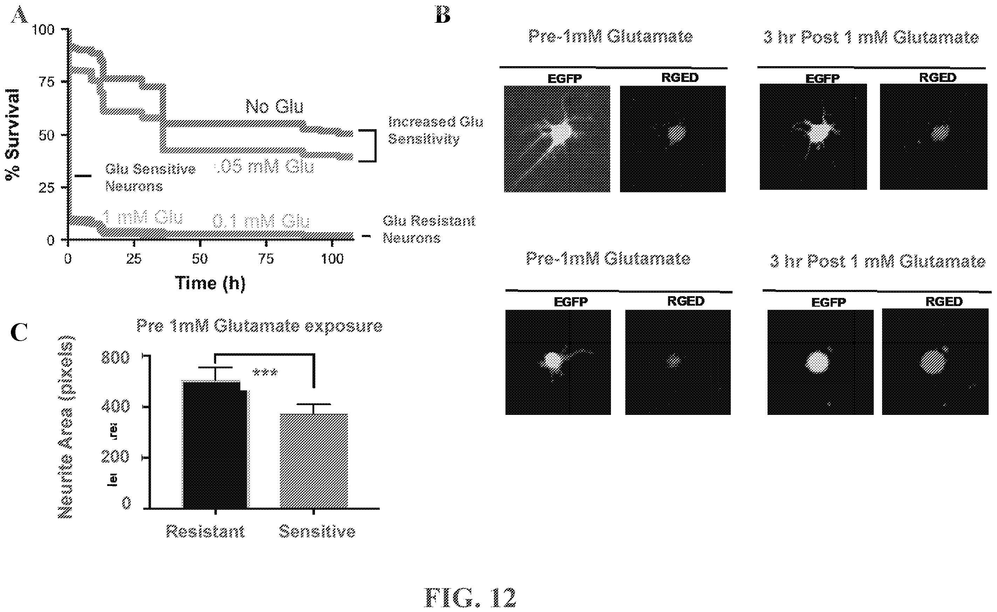

Briefly, neurons expressing RGEDI were exposed to the neurotransmitter glutamate. Within three hours, the glutamate killed many of the cells. FIG. 12A shows that rat primary cortical neurons after exposure to various amounts of glutamate showed different reactions. Using time lapse imaging, a subset of neurons could be identified that were resistant to glutamate, while another subset of neurons had increased sensitivity to glutamate as seen in FIGs. 12B and 12C. Furthermore, features of neurons such as neurite area were identified as associated with neurons that are resistant to high Glutamate exposure. These studies indicate that GEDIs can be used for screening for drug resistant or drug sensitive cells and identifying physiological characteristics of those cells that may underlie their sensitivity or resistance.

It is contemplated that GEDI can facilitate the next-generation of image analysis of cellular processes, such as neurodegeneration, with machine learning approaches. Supervised machine learning requires large, curated data sets to train models on, making robotic microscopy an ideal platform to provide both training and testing of data sets for machine learning approaches to understanding neurodegenerative diseases. Previous attempts at using machine learning approaches, however, have had disappointing results because of the inability to accurately and fully classify data sets due to the error involved in classifying dead cells, for example neurons, manually.

From the foregoing, it will be appreciated that specific embodiments of the invention have been described herein for purposes of illustration. Accordingly, the invention is not limited except as by the appended claims.

## Claims

1. An isolated nucleic acid comprising:

    a. a promoter;
    b. a nucleotide sequence of SEQ ID NO. 1 encoding a modified calcium binding motif or variants thereof that retain biological activity, wherein the modified calcium binding motif comprises at least one amino acid substitution selected from E31D, F92W, E104D, D133E, or any combination thereof, and encoding a first detectable marker in the form of a green fluorescent protein (GFP); and
    c. a nucleotide sequence encoding a second detectable marker distinguishable from the first detectable marker; wherein the nucleic acid does not encode an endoplasmic reticulum localization sequence, mitochondrial localization sequence, nuclear localization sequence or combination thereof.

2. The isolated nucleic acid of claim 1, wherein the promoter is a tissue specific promoter, optionally a neuron specific promoter.

3. The isolated nucleic acid of claim 1, wherein:
    the second detectable marker comprises a fluorescent label, preferably selected from green fluorescent protein (GFP), mApple, mTagBFP, or variants thereof that retain biological activity.

4. The isolated nucleic acid of claim 1 comprising a nucleotide sequence having at least 60%, at least 65%, at least 70%, at least 75%, at least 80%, at least 85%, at least 90%, at least 95%, at least 99% or 100% nucleotide sequence identity of SEQ ID NO: 6, SEQ ID NO: 7, SEQ ID NO: 8 or SEQ ID NO: 10, and which retains biological activity.

5. A vector comprising the nucleic acid of any one of the preceding claims.

6. The vector of claim 5, wherein the vector is an adenovirus, adeno-associated virus (AAV), lentivirus, retrovirus, or episomal vector.

7. An isolated cell comprising the isolated nucleic acid or vector of any one of the preceding claims, optionally wherein the cell is a neural cell, a muscle cell, or a cancer cell.

8. A non-human animal comprising the cell of claim 7.

9. An organotypic slice culture comprising the cell of claim 7.

10. A method for monitoring calcium flux in a cell comprising the isolated nucleic acid as in any of claims 1-4 comprising:

   a. measuring a signal from the first detectable marker to generate a first signal intensity;
   b. measuring a signal from the second detectable marker to generate a second signal intensity; and
   c. comparing the first signal intensity to the second signal intensity to generate a signal intensity ratio;

   optionally, wherein the measuring of step (a), step (b), or both is performed using confocal microscopy;
   optionally, wherein the method is a high-throughput method; or
   optionally, wherein the method is an automated method.

11. A method for monitoring cell death in a cell comprising the isolated nucleic acid as in any of claims 1-4 comprising:

   a. measuring a signal from the first detectable marker to generate a first signal intensity;
   b. measuring a signal from the second detectable marker to generate a second signal intensity;
   c. comparing the first signal intensity to the second signal intensity to generate a signal intensity ratio; and
   d. determining whether the cell is dead or alive based on the signal intensity ratio;

   optionally, wherein the signal intensity ratio of greater than 0.05 indicates the cell is dead;
   optionally, wherein the signal intensity ratio is determined using the formula:

   *signal intensity ratio* = ([(*GEDI dead ratio*) - (*GEDI live ratio*)] * 0.25) + [*avg. GEDI live*];

   optionally, wherein cell death is apoptosis, necrosis, autophagy, or necroptosis; or
   optionally, wherein cell death is determined when $Ca^{2+}$ levels inside the cell are equivalent to $Ca^{2+}$ levels outside the cell.

12. A method of screening for an agent that is capable of causing an intracellular calcium concentration fluctuation in a cell comprising:

   a. contacting a cell comprising the isolated nucleic acid as in any of claims 1-4 with the agent;
   b. measuring a signal from the first detectable marker to generate a first signal intensity;
   c. measuring a signal from the second detectable marker to generate a second signal intensity;
   d. comparing the first signal intensity to the second signal intensity to generate a signal intensity ratio; and
   e. determining whether the agent is capable causing an intracellular calcium concentration fluctuation based the signal intensity ratio.

**Patentansprüche**

1. Isolierte Nukleinsäure, die Folgendes umfasst:

   a. einen Promotor;
   b. eine Nukleotidsequenz von SEQ ID NO. 1, die ein modifiziertes Calciumbindungsmotiv oder biologische Aktivität beibehaltende Varianten codiert, wobei das modifizierte Calciumbindungsmotiv mindestens eine Aminosäuresubstitution umfasst, die aus E31D, F92W, E104D, D133E oder einer beliebigen Kombination davon ausgewählt ist und einen ersten nachweisbaren Marker in Form eines grün fluoreszierenden Proteins (GFP) codiert; und
   c. eine Nukleotidsequenz, die einen zweiten nachweisbaren Marker codiert, der von dem ersten nachweisbaren Marker unterscheidbar ist;

   wobei die Nukleinsäure keine endoplasmatische Retikulum-Lokalisierungssequenz, mitochondriale Lokalisierungssequenz, nukleare Lokalisierungssequenz oder eine Kombination davon codiert.

2. Isolierte Nukleinsäure nach Anspruch 1, wobei der Promotor ein gewebespezifischer Promotor, optional ein neuronenspezifischer Promotor, ist.

3. Isolierte Nukleinsäure nach Anspruch 1, wobei:
der zweite nachweisbare Marker eine Fluoreszenzmarkierung umfasst, die vorzugsweise aus grün fluoreszierendem Protein (GFP), mApple, mTagBFP oder biologische Aktivität beibehaltenden Varianten davon ausgewählt ist.

4. Isolierte Nukleinsäure nach Anspruch 1, die eine Nukleotidsequenz mit mindestens 60 %, mindestens 65 %, mindestens 70 %, mindestens 75 %, mindestens 80 %, mindestens 85 %, mindestens 90 %, mindestens 95 %, mindestens 99 % oder 100 % Nukleotidsequenzidentität mit SEQ ID NO: 6, SEQ ID NO: 7, SEQ ID NO: 8 oder SEQ ID NO: 10 aufweist und die biologische Aktivität beibehält.

5. Vektor, der die Nukleinsäure nach einem der vorherigen Ansprüche umfasst.

6. Vektor nach Anspruch 5, wobei der Vektor ein Adenovirus, ein Adeno-assoziiertes Virus (AAV), ein Lentivirus, ein Retrovirus oder ein episomaler Vektor ist.

7. Isolierte Zelle, die die isolierte Nukleinsäure oder den Vektor nach einem der vorherigen Ansprüche umfasst, wobei die Zelle optional eine Nervenzelle, eine Muskelzelle oder eine Krebszelle ist.

8. Nichthumanes Tier, das die Zelle nach Anspruch 7 umfasst.

9. Organotypische Scheibenkultur, die die Zelle nach Anspruch 7 umfasst.

10. Verfahren zum Überwachen des Calciumflusses in einer Zelle, das die isolierte Nukleinsäure nach einem der Ansprüche 1 bis 4 umfasst, das Folgendes beinhaltet:

a. Messen eines Signals von dem ersten nachweisbaren Marker, um eine erste Signalintensität zu erzeugen;
b. Messen eines Signals von dem zweiten nachweisbaren Marker, um eine zweite Signalintensität zu erzeugen; und
c. Vergleichen der ersten Signalintensität mit der zweiten Signalintensität, um ein Signalintensitätsverhältnis zu erzeugen;

wobei optional die Messung von Schritt (a), Schritt (b) oder beiden mittels Konfokalmikroskopie durchgeführt wird;
wobei das Verfahren optional ein Hochdurchsatzverfahren ist; oder
wobei das Verfahren optional ein automatisiertes Verfahren ist.

11. Verfahren zum Überwachen von Zelltod in einer Zelle, die die isolierte Nukleinsäure nach einem der Ansprüche 1-4 umfasst, das Folgendes beinhaltet:

a. Messen eines Signals von dem ersten nachweisbaren Marker, um eine erste Signalintensität zu erzeugen;
b. Messen eines Signals von dem zweiten nachweisbaren Marker, um eine zweite Signalintensität zu erzeugen;
c. Vergleichen der ersten Signalintensität mit der zweiten Signalintensität, um ein Signalintensitätsverhältnis zu erzeugen; und
d. Feststellen, ob die Zelle tot oder lebendig ist, auf der Basis des Signalintensitätsverhältnisses;

wobei optional ein Signalintensitätsverhältnis von mehr als 0,05 anzeigt, dass die Zelle tot ist;
wobei optional das Signalintensitätsverhältnis anhand der folgenden Formel bestimmt wird:

*Signalintensitätsverhältnis - ([(GEDI Totverhältnis) - GEDI Lebendverhältnis)]\*0,25) + [durchschn. GEDI lebend];*

wobei optional Zelltod Apoptose, Nekrose, Autophagie oder Nekroptose ist; oder
wobei optional Zelltod festgestellt wird, wenn $Ca^{2+}$-Konzentrationen innerhalb der Zelle $Ca^{2+}$-Konzentrationen außerhalb der Zelle entsprechen.

**12.** Verfahren zum Screenen nach einem Agens, das eine intrazelluläre Calciumkonzentrationsfluktuation in einer Zelle verursachen kann, das Folgendes beinhaltet:

a. In-Kontakt-Bringen einer die isolierte Nukleinsäure nach einem der Ansprüche 1-4 umfassenden Zelle mit dem Agens;
b. Messen eines Signals von dem ersten nachweisbaren Marker, um eine erste Signalintensität zu erzeugen;
c. Messen eines Signals von dem zweiten nachweisbaren Marker, um eine zweite Signalintensität zu erzeugen;
d. Vergleichen der ersten Signalintensität mit der zweiten Signalintensität, um ein Signalintensitätsverhältnis zu erzeugen; und
e. Feststellen, ob der Wirkstoff eine intrazelluläre Calciumkonzentrationsfluktuation bewirken kann, auf der Basis des Signalintensitätsverhältnisses.

**Revendications**

**1.** Acide nucléique isolé comprenant :

a. un promoteur ;
b. une séquence nucléotidique de SEQ ID No. 1 codant pour un motif modifié liant le calcium ou des variantes de celui-ci qui conservent une activité biologique, dans lequel le motif modifié liant le calcium comprend au moins une substitution d'acide aminé choisie dans E31D, F92W, E104D, D133E, ou toute combinaison de celles-ci, et codant pour un premier marqueur détectable sous la forme d'une protéine fluorescente verte (GFP) ; et
c. une séquence nucléotidique codant pour un second marqueur détectable pouvant être distingué du premier marqueur détectable ;
dans lequel l'acide nucléique ne code pas pour une séquence de localisation du réticulum endoplasmique, une séquence de localisation mitochondriale, une séquence de localisation nucléaire ou une combinaison de celles-ci.

**2.** Acide nucléique isolé selon la revendication 1, dans lequel le promoteur est un promoteur à spécificité tissulaire, facultativement un promoteur à spécificité neuronale.

**3.** Acide nucléique isolé selon la revendication 1, dans lequel :
le second marqueur détectable comprend une étiquette fluorescente, de préférence choisie dans protéine fluorescente verte (GFP), mApple, mTagBFP, ou des variantes de celles-ci qui conservent une activité biologique.

**4.** Acide nucléique isolé selon la revendication 1 comprenant une séquence nucléotidique présentant au moins 60 %, au moins 65 %, au moins 70 %, au moins 75 %, au moins 80 %, au moins 85 %, au moins 90 %, au moins 95 %, au moins 99 % ou 100 % d'identité de séquences nucléotidiques de SEQ ID No. 6, SEQ ID No. 7, SEQ ID No. 8 ou SEQ ID No. 10, et qui conserve une activité biologique.

**5.** Vecteur comprenant l'acide nucléique selon l'une quelconque des revendications précédentes.

**6.** Vecteur selon la revendication 5, dans lequel le vecteur est un adénovirus, un virus adéno-associé (AAV), un lentivirus, un rétrovirus ou un vecteur épisomal.

**7.** Cellule isolée comprenant l'acide nucléique isolé ou un vecteur selon l'une quelconque des revendications précédentes, facultativement dans laquelle la cellule est une cellule neuronale, une cellule musculaire ou une cellule cancéreuse.

**8.** Animal non humain comprenant la cellule selon la revendication 7.

**9.** Culture de tranches organotypiques comprenant la cellule selon la revendication 7.

**10.** Procédé de surveillance du flux de calcium dans une cellule comprenant l'acide nucléique isolé selon l'une quelconque des revendications 1 à 4 comprenant :

a. la mesure d'un signal provenant du premier marqueur détectable pour générer une première intensité de signal ;

b. la mesure d'un signal provenant du second marqueur détectable pour générer une seconde intensité de signal ; et

c. la comparaison de la première intensité de signal à la seconde intensité de signal pour générer un rapport d'intensités de signal ;

facultativement, dans lequel la mesure de l'étape (a), de l'étape (b), ou des deux est réalisée en utilisant une microscopie confocale ;
facultativement, dans lequel le procédé est un procédé à haut rendement ; ou facultativement, dans lequel le procédé est un procédé automatisé.

11. Procédé de surveillance de la mort cellulaire dans une cellule comprenant l'acide nucléique isolé selon l'une quelconque des revendications 1 à 4 comprenant :

a. la mesure d'un signal provenant du premier marqueur détectable pour générer une première intensité de signal ;
b. la mesure d'un signal provenant du second marqueur détectable pour générer une seconde intensité de signal ;
c. la comparaison de la première intensité de signal à la seconde intensité de signal pour générer un rapport d'intensités de signal ; et
d. la détermination de savoir si la cellule est morte ou vivante sur la base du rapport d'intensités de signal ;

facultativement, dans lequel le rapport d'intensités de signal supérieur à 0,05 indique que la cellule est morte ;
facultativement, dans lequel le rapport d'intensités de signal est déterminé en utilisant la formule :

rapport d'intensités de signal = ([(rapport mortes GEDI) - (rapport vivantes GEDI)] * 0,25) + [moy. vivantes GEDI] ;

facultativement, dans lequel la mort cellulaire est une apoptose, une nécrose, une autophagie, ou une nécroptose ; ou
facultativement, dans lequel la mort cellulaire est déterminée lorsque les niveaux de $Ca^{2+}$ à l'intérieur de la cellule sont équivalents aux niveaux de $Ca^{2+}$ à l'extérieur de la cellule.

12. Procédé de criblage d'un agent qui est apte à entraîner une fluctuation de concentration de calcium intracellulaire dans une cellule comprenant :

a. la mise en contact d'une cellule comprenant l'acide nucléique isolé selon l'une quelconque des revendications 1 à 4 avec l'agent ;
b. la mesure d'un signal provenant du premier marqueur détectable pour générer une première intensité de signal ;
c. la mesure d'un signal provenant du second marqueur détectable pour générer une seconde intensité de signal ;
d. la comparaison de la première intensité de signal à la seconde intensité de signal pour générer un rapport d'intensités de signal ; et
e. la détermination de savoir si l'agent est apte à entraîner une fluctuation de concentration de calcium intracellulaire sur la base du rapport d'intensités de signal.

# FIG. 1

FIG. 1, *cont.*

FIG. 2

# FIG. 2, *cont.*

FIG. 2, *cont.*

G

FIG. 3

# FIG. 4

A

B

FIG. 5

| Method | HR | p value | Low95 | Upp95 | # neurons |
|---|---|---|---|---|---|
| Manual | 1.398 | 3.69E-04 | 1.163 | 1.681 | 334/315 |
| Automated | 1.017 | 0.78 | 0.903 | 1.146 | 749/712 |
| RGEDI Intensity | 1.367 | 7.90E-04 | 1.139 | 1.64 | 344/301 |

— HttEx1-Q97
— HttEx1-Q25

**FIG. 5,** *cont.*

EP 3 529 622 B1

# FIG. 6

FIG. 6, *cont.*

C

FIG. 7

FIG. 7, *cont.*

A

Cytoplasm

ER

Extracellular

GCaMP6f-mRuby

RGEDI

RGEDI

GGEDI

GEM-GEDI

0.01    0.1    1    10    100    1000    10,000

Ca$^{2+}$ concentration (µM)

B

| Construct | Death Indicator Color | Morphology Marker Emission Color and protein | GEDI Predicted K$_d$ | GEDI Predicted Hill Coefficient |
|---|---|---|---|---|
| GCaMP6f-P2A-mRuby | Green | Red-mRuby | 0.375 µM$^x$ | 2.27±0.1 µM$^x$ |
| RGEDI-P2A-EGFP | Red | Green- EGFP | 565±58 µM* | 1.70±0.04 µM* |
| RGEDInls-P2A-EGFPnls | Red | Green- EGFP | 565±58 µM* | 1.70±0.04 µM* |
| GGEDI-P2A-mApple | Green | Red- mApple | 672±4 µM* | 1.95±0.07 µM* |
| RGEDI-P2A-3xtagBFP2 | Red | Blue- TagBFP2 | 565±58 µM* | 1.70±0.04 µM* |
| GEM-CEPIA-P2A-EGFP | Green->Blue | Far Red-mKate2 | 558±14 µM* | 1.37±0.01 µM* |

FIG. 8

FIG. 9

FIG. 10

C

| | 0 hours | 24 hours | 48 hours | 72 hours |

RGEDI

GCaMP6

3xBFP

*

Overlay

**FIG. 10,** *cont.*

# FIG. 11

A

B

FIG. 11, *cont.*

C

FIG. 12

# FIG. 13

A

FIG. 13, *cont.*

**B**

| | 0 hours | 24 hours | 48 hours |
|---|---|---|---|

RGEDI

Overlay

RGEDI

NTR-mVenus

BFP

# FIG. 14

A

FIG. 14, *cont.*

# FIG. 15

## cfGCaMP2 variants

| Mutated amino acids | Dynamic range | Hill coeff | $K_d$ (µM) |
|---|---|---|---|
| Original (CEPIA2mr) | 5.1 | 2.5 | 0.67 |
| ■ Substitutions used in previously reported ER Ca$^{++}$ indicators | | | |
| E11K/E84R/E87K (D1ER) | 2.6 | 0.9 | 14.5 |
| E31Q(YC4er) | 2.5 | 0.5 | 64.9 |
| E104Q(YC3er) | 3.4 | 3.7 | 1.2 |
| ■ Single substitution at -Z position | | | |
| E31D (Split-YC7.3er, CEPIA3mt) | 5.0 | 1.5 | 14.5 |
| E67D | 4.7 | 1.4 | 9.2 |
| E104D | 3.8 | 4.2 | 0.99 |
| E140D | 4.4 | 3.7 | 2.1 |
| ■ Double substitution at -Z positions | | | |
| E31D/E67D | 2.6 | 1.3 | 470 |
| E31D/E104D | 4.1 | 1.5 | 20.7 |
| E31D/E140D | 4.7 | 2.0 | 23.5 |
| E67D/E140D | 4.7 | 2.1 | 23.9 |
| E104D/E140D | 4.2 | 1.2 | 24.4 |
| ■ Triple substitutions at -Z positions | | | |
| E31D/E104D/E140D | 3.2 | 1.9 | 135 |
| E67D/E104D/E140D | 3.6 | 2.2 | 129 |
| ■ Substitutions at F92W and/or D133E | | | |
| F92W | 4.5 | 3.3 | .090 |
| D133E | 4.3 | 4.7 | 2.1 |
| F92W/D133D | 4.6 | 3.0 | 10.3 |
| ■ F92W/D133D and single substitutions at -Z positions | | | |
| E31D/F92W/D133E (CEPIA4mt) | 4.9 | 1.7 | 90.2 |
| E67D/F92W/D133E | 4.9 | 2.3 | 75.2 |
| F92W/E104D/D133E | 3.5 | 1.3 | 130 |
| F92W/D133E/E140D | 4.5 | 0.9 | 67.4 |

## R-GECO1 variants with cfGCaMP2 CaM

| Mutated amino acids | Dynamic range | Hill coeff | $K_d$ (µM) |
|---|---|---|---|
| E31D/E67d | 17.6 | 1.2 | 152 |
| E31D/F92W/D133D | 21.9 | 2.3 | 16.6 |
| E31D/E104D/D133E | 19.8 | 2.7 | 26.3 |
| E104D/D133E/E140D | 12.5 | 1.4 | 50.8 |
| E31D/E67D/F92W/D113D | 17.2 | 1.2 | 211 |
| E31D/F922/E104D/D133E | 16.9 | 2.3 | 70.9 |
| E67D/F92W/D133E/E140D | 17.6 | 2.1 | 20.0 |
| E92W/E104D/D133E/140D | 14.9 | 1.0 | 123 |
| E31D/E67D/F92W/E104D/ D1332 )R-CEPIA1er) | 8.8 | 1.7 | 565 |

## G-GECO1.1 variants with cfGCaMP2 CaM

| Mutated amino acids | Dynamic range | Hill coeff | $K_d$ (µM) |
|---|---|---|---|
| E31D/F92W/D133E | 10.2 | 1.5 | 209 |
| E31D/F92W/E104D/ D133E (G-CEPIA1er) | 4.7 | 1.9 | 672 |
| E31D/E67D/F92W/E104D/ D133D | 3.8 | 0.5 | 3,860 |

## GEM-GECO1 variants with cfGCaMP2 CaM

| Mutated amino acids | | | |
|---|---|---|---|
| E31D/F92W/E104D/D133E | 1.1 | n.d. | n.d. |
| E31D/E67D/F92W/E104D/ D133E | 1.2 | n.d. | n.d. |

# FIG. 15, *cont.*

■ F92W/D133E and double substitutions at -Z positions

| | | | |
|---|---|---|---|
| E31D/F92W/E104D/D133E (CEPIA1er) | 4.2 | 1.3 | 368 |
| E31D/F92W/D133E/E140D | 3.5 | 1.8 | 411 |
| E67D/F92W/D104D/D133E | 4.1 | 1.9 | 344 |
| E67D/F92W/D133E/D140D | 4.4 | 1.7 | 276 |
| F92W/E104D/D133E/E140D[+] | 1.3,2.7 | 1.9,1.4 | 1,540 |

■ D133E and single substitutions at -Z positions

| | | | |
|---|---|---|---|
| E31D/D133E | 5.0 | 2.2 | 33.2 |
| E67D/D133E | 5.0 | 2.4 | 31.1 |
| E104D/D133E | 3.5 | 1.2 | 43.8 |
| D133E/E140D | 4.5 | 2.2 | 8.2 |

■ D133E and double substitutions at -Z positions

| | | | |
|---|---|---|---|
| E31D/E104D/D133E | 4.2 | 2.0 | 201 |
| E31D/D133E/E140D | 4.7 | 1.8 | 92.2 |
| E67D/E104D/D133E | 4.1 | 2.2 | 154 |
| E67D/D133E/E140D | 4.4 | 1.9 | 93.4 |
| D104D/D133E/E140D[+] | 1.3,3.1 | 1.7,1.3 | 4.8,661 |

■ Other substitutions

| | | | |
|---|---|---|---|
| E11K | 2.1 | 3.6 | 1.4 |
| E8AR/E87K | 3.3 | 2.8 | 1.2 |
| E31A | 1.9 | 0.8 | 15.2 |
| E31A/D133E | 2.2 | 0.6 | 109 |
| E67D/E104Q | 4.2 | 1.4 | 43.7 |
| E104D/E140D | 3.1 | 0.8 | 23.8 |
| E31Q/F92W/D133E | 2.1 | 0.3 | 1,730 |
| F92W/E104Q/D133E[+] | 1.5,1.4 | 2.0,0.9 | 4.3,6,100 |
| E31D/F92W/E104Q/D133E | 1.8 | 0.6 | 13,400 |
| E67D/F92W/E104Q/D133E | 2.4 | 0.7 | 2,330 |
| F92W/S101D/D133E | 5.1 | 3.2 | 7.0 |
| F92W/D95N/N97D/D133E | 4.9 | 3.2 | 8.2 |
| F92W/D95N/S101D/D133E | 4.3 | 2.7 | 11.1 |
| E31D/L36M | 4.0 | 1.1 | 22.4 |
| E31D/L36M/E67D | 2.8 | 1.2 | 873 |
| E31D/L36M/E104D | 4.0 | 1.5 | 25.0 |
| E31D/L36M/E104D | 4.9 | 2.0 | 26.1 |
| E31D/L36M/E104Q | 3.8 | 1.5 | 45.2 |
| T26G/E31D/L36M | 4.6 | 0.9 | 2.4 |
| E31D/L36M/Q41L | 3.9 | 1.3 | 15.1 |
| E31D/L36M/K75I | 3.7 | 1.2 | 9.2 |
| E31D/L36M/Q41L/K75I | 3.1 | 1.1 | 8.9 |

## GEM-GECO1 variants with original CaM

| Mutated amino acids | Dynamic range | Hill coeff. | $K_d(\mu M)$ |
|---|---|---|---|
| Original | 67.5 | 2.6 | 0.31 |
| E31D | 23.7 | 1.2 | 7.2 |
| E31D/E67D | 13.0 | 1.2 | 97.1 |
| E31D/D133E | 41.3 | 2.4 | 36.4 |
| E31D/E140D | 43.6 | 2.2 | 24.2 |
| E31D/F92W/D133E | 11.2 | 1.9 | 90.0 |
| E31D/E104D/D133E | 20.8 | 1.7 | 89.7 |
| E31D/D133E/E140D | 24.8 | 1.6 | 147 |
| E31D/F92W/E104D/D133E | 8.1 | 2.1 | 225 |
| E31D/F92W/D133E/E140D (GEM-CEPIA1er) | 21.7 | 1.4 | 558 |
| E31D/E104D/D133E/E140D | 34.8 | 1.5 | 483 |
| E31D/E67D/F92W/D133E/ E140D | 1.4 | 1.1 | 602 |
| E31D/F92W/E104D/D133E/ E140D | 2.3 | 1.0 | 2,010 |

# FIG. 16

# FIG. 17

FIG. 18

FIG. 19

FIG. 20

EP 3 529 622 B1

FIG. 21

# FIG. 22

## REFERENCES CITED IN THE DESCRIPTION

**Patent documents cited in the description**

- US 62409941
- US 20150226755 A
- US 4683195 A
- US 6333195 B
- US 5449614 A
- US 6995009 B
- US 5834256 A
- US 6910434 B
- US 5591624 A
- US 5817491 A
- US 7070994 B
- US 6175057 B
- US 6180849 B
- US 6133502 A

**Non-patent literature cited in the description**

- **SUZUKI et al.** Imaging intraorganellar Ca2+ at subcellular resolution using CEPIA. *Nat. Commun.*, 2014, vol. 5, 4153
- **ARRASATE M. et al.** *Nature*, 2014, vol. 431, 805-810
- Molecular Cloning: A Laboratory Manual. 2001
- Current Protocols in Molecular Biology. 2007
- Methods in Enzymology. Academic Press, Inc., N.Y.
- **MACPHERSON et al.** PCR 1: A Practical Approach. IRL Press at Oxford University Press, 1991
- **MACPHERSON et al.** PCR 2: A Practical Approach. 1995
- Antibodies, A Laboratory Manual. 1999
- **FRESHNEY.** Culture of Animal Cells: A Manual of Basic Technique. 2005
- Oligonucleotide Synthesis. 1984
- Nucleic Acid Hybridization. 1984
- **ANDERSON.** Nucleic Acid Hybridization. 1999
- Transcription and Translation. 1984
- Immobilized Cells and Enzymes. IRL Press, 1986
- **PERBAL.** A Practical Guide to Molecular Cloning. 1984
- Gene Transfer Vectors for Mammalian Cells. Cold Spring Harbor Laboratory, 1987
- Gene Transfer and Expression in Mammalian Cells. 2003
- Immunochemical Methods in Cell and Molecular Biology. Academic Press, 1987
- Weir's Handbook of Experimental Immunology; Manipulating the Mouse Embryo: A Laboratory Manual. Cold Spring Harbor Laboratory Press, 1996
- **SOHAIL.** Gene Silencing by RNA Interference: Technology and Application. CRC Press, 2004
- **JOHNSTON et al.** *Journal of Virology*, 1999, vol. 73 (6), 4991-5000
- **NEGRE et al.** *Current Topics in Microbiology and Immunology*, 2002, vol. 261, 53-74
- **NALDINI et al.** *Science*, 1996, vol. 272, 263-267
- **YEE et al.** *Methods Cell Biol.*, 1994, 99-112
- **MORITA et al.** *Gene Therapy*, 2000, vol. 7 (12), 1063-1066
- **ONISHI et al.** *Experimental Hematology*, 1996, vol. 24, 324-329
- **MIYOSHI et al.** *J. Virol.*, 1998, vol. 72 (10), 8150-8157
- **RIVIERE et al.** *Proc. Natl. Acad. Sci.*, 1995, vol. 92, 6733-6737
- **KIMATURA et al.** *Experimental Hematology*, 2003, vol. 31, 1007-1014
- **MFG RIVIERE et al.** *Proc. Natl. Acad. Sci.*, 1995, vol. 92, 6733-6737
- **MORGENSTERN et al.** *Nucleic Acids Research*, 1990, vol. 18, 3587-3596
- **CLG MIYOSHI et al.** *J. Vir.*, 1998, vol. 72, 8150-8157
- **KANEGAE et al.** *Nucleic Acids Research*, 1995, vol. 23, 3816-3821
- **FELGNER et al.** *Proc. Natl. Acad. Sci.*, 1987, vol. 84, 7413-7417
- **BEVIS et al.** *Nat. Biotechnol.*, 2002, vol. 20 (11), 83-87
- **WAGNER et al.** *Proc. Nat. Acad. Sci. USA*, 1981, vol. 78, 5016-5020
- **STEWART et al.** *Science*, 1982, vol. 217, 1046-1048
- **CONSTANTINI et al.** *Nature*, 1981, vol. 294, 92-94

- **LACY et al.** *Cell*, 1983, vol. 34, 343-358
- **MCKNIGHT et al.** *Cell*, 1983, vol. 34, 335-341
- **BRINSTAR et al.** *Nature*, 1983, vol. 306, 332-336
- **PALMITER et al.** *Nature*, 1982, vol. 300, 611-615
- **PALMITER et al.** *Cell*, 1982, vol. 29, 701-710
- **PALMITER et al.** *Science*, 1983, vol. 222, 809-814
- **TAKAHASHI et al.** *Cell*, 2007, vol. 131 (5), 861-872
- **KIM et al.** *Proc. Natl. Acad. Sci.*, 2011, vol. 108 (19), 7838-7843
- **SELL**. *Stem Cells Handbook*, 2013
- **KANG**. *Sci. Rep.*, 2016, vol. 6, 28798
- **SEIL**. *Review in Neuroscience*, 1979, vol. 4, 105-177
- **GAHWILER**. *J Neurosci Meth*, 1981, vol. 4, 329-342
- **GAHWILER**. *Neuroscience*, 1984, vol. 11, 751-760
- **GAHWILER**. *Trends Neurosci*, 1988, vol. 11, 484-490
- **STOPPINI et al.** *J Neurosci Methods*, 1991, vol. 37, 173-182
- **ARRASATE et al.** *Nature*, 2014, vol. 431, 805-810
- **ARRASATE M. et al.** *Proc Natl Acad Sci U S A.*, 2005, vol. 102 (10), 3840-5